# EUROPEAN PATENT SPECIFICATION

(11) **EP 3 962 543 B1**
(45) Date of publication and mention of the grant of the patent: **13.03.2024**
(21) Application number: 20721632.6
(22) Date of filing: 30.04.2020
(51) Int. Cl.: A61L 15/28, A61L 15/46, A61L 15/60, A61L 27/20, A61L 27/52, A61L 27/54, A61L 29/04, A61L 29/14, A61L 29/16, A61L 31/04, A61L 31/14, A61L 31/16

(54) **HYALURONIC ACID HYDROGELS WITH PROLONGED ANTIMICROBIAL ACTIVITY**
HYALURONSÄUREHYDROGELE MIT VERLÄNGERTER ANTIMIKROBIELLER WIRKUNG
HYDROGELS D'ACIDE HYALURONIQUE AVEC UNE ACTIVITÉ ANTIMICROBIENNE PROLONGÉE

(30) Priority: 02.05.2019 EP 19305562
(43) Date of publication of application: 09.03.2022
(73) Proprietor: Institut National de la Santé et de la Recherche Médicale (INSERM), 75013 Paris (FR); Université de Strasbourg, 67000 Strasbourg (FR); Spartha Medical, 67000 Strasbourg (FR)
(72) Inventor: LAVALLE, Philippe, 67370 WINTZENHEIM (FR); CALLIGARO, Cynthia, 67100 STRASBOURG (FR); GRIBOVA, Varvara, 67100 STRASBOURG (FR); TALLET, Lorène, 67000 STRASBOURG (FR); VRANA, Nihal Engin, 67100 STRASBOURG (FR)
(74) Representative: Lavoix
(86) International application number: PCT/EP2020/062137
(87) International publication number: WO 2020/221896

(56) References cited:
- EP-A1- 3 241 570
- LEQUEUX ISABELLE ET AL: "Addition of antimicrobial properties to hyaluronic acid by grafting of antimicrobial peptide", EUROPEAN POLYMER JOURNAL, PERGAMON PRESS LTD. OXFORD, GB, vol. 51, 20 November 2013 (2013-11-20), pages 182-190, XP028810221, ISSN: 0014-3057, DOI: 10.1016/J.EURPOLYMJ.2013.11.012
- KHUNMANEE S ET AL: "Crosslinking method of hyaluronic-based hydrogel for biomedical applications", JOURNAL OF TISSUE ENGINEERING,, vol. 8, 1 January 2017 (2017-01-01), pages 1-16, XP002791499,
- SRINIVAS SUDA ET AL: "Investigating the importance of charged residues in lantibiotics", BIOENGINEERED BUGS, vol. 1, no. 5, 1 September 2010 (2010-09-01), pages 345-351, XP055634431, US ISSN: 1949-1018, DOI: 10.4161/bbug.1.5.12353

## Description

The present invention concerns hydrogels with antimicrobial activity.

Implantation of biomedical devices is often followed by excessive immune response to the implant, as well as bacterial, yeast and fungal infections. Inflammation and infection may seriously affect implant functionalities and even lead to their failure.

There is thus an important need for solutions for avoiding such infections after implantation of biomedical devices.

Hydrogels have several unique characteristic properties, including their similarity to tissue extracellular matrix, support for cell proliferation and migration, controlled released of drugs or growth factors, minimal mechanical irritation to surrounding tissue, and nutrient diffusion, that support the viability and proliferation of cells. Accordingly, hydrogels are promising materials in the field of tissue engineering.

Hyaluronic acid is widely used to prepare biomaterials for tissue engineering because it yields highly reproducing and affordable biomaterials.

Addition of antimicrobial properties to hyaluronic acid by grafting of antimicrobial peptide (Lequeux Isabelle; Ducasse Emmanuel; Jouenne Thierry; Thebault Pascal; European Polymer Journal 51, (2014) 192-200) discloses an antimicrobial peptide, nisin, attached to hyaluronic acid (HA) in the form of a gel. The HA was solubilized in PBS. Coupling agents EDC/NHS were added. Previously prepared nisin hydrochloric solution was added and the pH adjusted to result in the modified HA.

However, HA hydrogels as such do not have any activity against possible infections. Furthermore, the use of HA in tissue engineering has been associated with many drawbacks, including short half-life, fast turnover, which affect the interest of its use in tissue engineering.

There is thus an important need for new materials useful for implantation of biomedical devices or tissue engineering, which can be conveniently manipulated by the physician, do not display a too fast turnover and have antimicrobial activity while remain safe for the patient to be treated.

The present invention meets this need.

The present invention arises from the unexpected finding by the inventors that it is possible to develop HA hydrogel loaded with polyarginine, which provide a long lasting antimicrobial effect and can be easily deposited onto wound dressings and mesh prosthesis to prevent infections, thus improving tissue regeneration and/or implant integration.

The present invention thus concerns a hydrogel comprising hyaluronic acid (HA) or a derivative thereof, loaded with at least one positively charged antimicrobial peptide, without said peptide being covalently linked to the hydrogel, wherein said HA or derivative thereof is cross-linked with a cross-linking agent at the level of its hydroxyl moieties while the carboxyl moieties of HA or derivative thereof remain free and said HA or derivative thereof remains negatively charged, wherein the derivatives of hyaluronic acid are selected from the group consisting of: HA modified with an aldehyde group, amine-modified hyaluronic acid, HA containing photoreactive vinylbenzyl groups, HA modified with a methacrylate group, HA conjugated to Tyramine, and HA conjugated to catechol.

Another object of the invention concerns a method for preparing a hydrogel according to the invention, wherein said method comprises the following steps:
(a) mixing, in basic conditions, hyaluronic acid (HA) or a derivative thereof with a cross-linking agent which cross-links HA at the level of its hydroxyl moieties while the carboxyl moieties of HA or derivative thereof remain free and said HA or derivative thereof remains negatively charged, wherein the derivatives of hyaluronic acid are selected from the group consisting of: HA modified with an aldehyde group, amine-modified hyaluronic acid, HA containing photoreactive vinylbenzyl groups, HA modified with a methacrylate group, HA conjugated to Tyramine, and HA conjugated to catechol.
(b) depositing the mixture on a support and incubating it for 48 h to 72 h at room temperature to obtain a hydrogel,
(c) recovering the hydrogel formed at step (b),
(d) incubating said hydrogel in an aqueous buffer in conditions enabling the withdrawal of cross-linking agent residues and the hydrogel to swell,
(e) loading the hydrogel obtained at step (d) with at least one positively charged antimicrobial peptide, without said peptide being covalently linked to the hydrogel, and
(f) recovering the loaded hydrogel obtained at step (e).

Still another object of the invention relates to an hydrogel according to the invention, likely to be obtained by the method of preparation of the invention.

The present invention further concerns a medical device comprising a hydrogel according to the invention.

### Detailed description of the invention

### Hyaluronic acid hydrogel

As used herein, the term 'hydrogel" refers to a chemically cross-linked hydrogel which retains water.

The hydrogel of the invention comprises hyaluronic acid (HA) or a derivative thereof.

As used herein, the term "hyaluronic acid (HA)" also known as Hyaluronan is a linear (unbranched) polysaccharide or non-sulfated glycosaminoglycan, composed of repeating disaccharide units of N-acetyl glucosamine and glucuronate (linked by β 1-3 and β 1-4 glycosidic bonds).

HA is typically of the following formula (3): 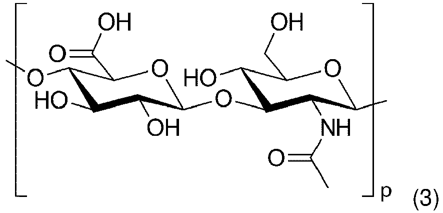 wherein p is an integer comprised between 2 and 25,000.

Hyaluronic acid (HA) is thus a negatively charged polymer (also called polyanion). Said negatively charged polymer therefore exists together with a counter ion in form of a salt. For sodium hyaluronate the counterion is sodium. Hyaluronic acid can be degraded by Hyaluronidase. The molecular weight (Mw) of hyaluronan represents an average of all the molecules in the population and thus represents the molecular Mass Average (Molecular Weight Average). Hyaluronic acid (HA) is available in a broad range of molecular weights.

"A type of hyaluronic acid" thus refers to a particular hyaluronic acid with a specific molecular weight.

In a particular embodiment, said hyaluronic acid is a hyaluronic acid having a molecular weight of between 150 kDa and 3000 kDa, in particular between 160 kDa and 2900 kDa, between 170 kDa and 2800 kDa, between 180 kDa and 2700 kDa, between 190 kDa and 2670 kDa, between 200 kDa and 2600 kDa, between 300 kDa and 2500 kDa, between 400 kDa and 2400 kDa, between 500 kDa and 2300 kDa, between 600 kDa and 2200 kDa, between 700kDa and 2100 kDa, between 750 kDa and 2000 kDa, between 760 kDa and 1900 kDa, between 770 kDa and 1800 kDa, between 780 kDa and 1700 kDa, between 790 kDa and 1600 kDa, between 800 kDa and 1500 kDa, between 805 kDa and 1400 kDa, between 810 kDa and 1300 kDa, between 815 kDa and 1200 kDa, between 820 kDa and 1100 kDa, between 821 kDa and 1000 kDa, between 822 kDa and 900 kDa, between 823 kDa and 850 kDa.

In one example, hyaluronic acid has a molecular weight of 150 kDa and is brought in form of Sodium Hyaluronate from Lifecore Biomed, USA.

In a preferred embodiment, said hyaluronic acid is a hyaluronic acid having a molecular weight of between 800 and 850 kDa.

In a preferred example, hyaluronic acid has a molecular weight of 823 kDa (referred to as HA⁸⁰⁰).

In another example, hyaluronic acid has a molecular weight of 2670 kDa (referred to as HA²⁷⁰⁰).

In a particular embodiment said hydrogel comprises more than one type of hyaluronic acid. Accordingly, in some embodiments the hyaluronic acid comprises at least two types of hyaluronic acid and one of the at least two types of hyaluronic acid has a molecular weight of between 800 and 850 kDa and the other one of the at least two types of hyaluronic acid has a molecular weight of between 100 and 2000 kDa, in particular a molecular weight of 150 kDa.

As used herein, the term "derivative of hyaluronic acid" herein refers to chemically modified hyaluronic acid. More particularly, a derivative of hyaluronic acid may refer to hyaluronic acid that has been chemically modified to introduce chemical groups or to conjugate HA with a chemical compound; which preferably enables cross-linking of HA with another compound or with another HA compound or derivative thereof, in particular a compound bearing an amine group.

According to the invention, the derivatives of hyaluronic acid are selected from the group consisting of: HA modified with an aldehyde group (referred to as HA-CHO or HA-Ald), amine-modified hyaluronic acid (referred to as HA-NH2), HA containing photoreactive vinylbenzyl groups (referred to as HA-VB), HA modified with a methacrylate group (referred to as methacrylated HA), HA conjugated to Tyramine (referred to as HA-Tyramine), and HA conjugated to catechol (referred to as HA-catechol).

In the hydrogel of the invention, said HA or derivative thereof is cross-linked with a cross-linking agent at the level of its hydroxyl moieties while the carboxyl moieties of HA or derivative thereof remain free and said HA or derivative thereof remains negatively charged.

By "cross-linking agent" is meant herein a reagent enabling the formation of covalent bonds or ionic bonds between two polymers, namely two HA molecules.

In the context of the invention, the cross-linking agent cross-link HA at the level of its hydroxyl moieties while the carboxyl moieties of HA remain free and HA remains negatively charged.

By "carboxyl moiety remaining free" is meant herein that the cross-linking agent does not induce the formation of a bond at the level of the carboxyl moieties of HA, which thus remain unmodified compared to their state before cross-linking.

Examples of cross-linking agents targeting hydroxyl groups are well-known from the skilled person and include butanediol diglycidyl ether (BDDE), divinyl sulfone (DVS) and cyanogen bromide, octeylsuccinic anhydride.

As will be understood by the skilled person, whether or not a cross-linking agent will induce the formation at a particular moiety may depend on the reaction conditions, in particular depend on a reaction in acid or basic conditions.

By "HA remaining negatively charged" is meant herein the global charge of HA after cross-linking is negative. In a particular embodiment, all the HA molecules remain negatively charged after cross-linking.

In a particular embodiment, said cross-linking agent is butanediol diglycidyl ether (BDDE).

HA cross-linked with BDDE is typically of the following formula (4)

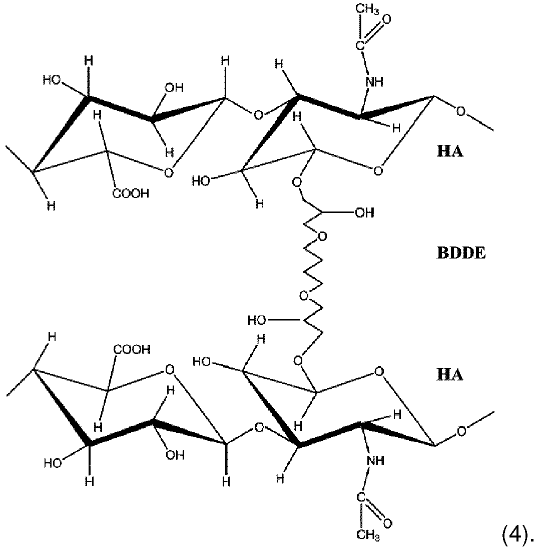

The hydrogel of the invention typically has a high cross-linking level.

### Positively charged antimicrobial peptide

The HA hydrogel of the invention is loaded with at least one positively charged antimicrobial peptide.

By "loaded" is meant herein that the HA hydrogel is impregnated, comprises and/or bears at least one positively charged antimicrobial peptide, without said peptide being covalently linked to the hydrogel. Said peptide can thus be naturally released from the hydrogel overtime.

By "antimicrobial peptide" is meant herein a peptide displaying antiseptical, antibiotic, antibacterial, antiviral, antifungal, antiprotozoal, and/or antiparasitic activity.

Preferably, the antimicrobial peptide is an antibacterial peptide.

As used herein, the term "antibacterial activity" encompasses bacteriostatic and/or bactericide activity.

In one embodiment the antibacterial activity and/or bacteriostatic activity is directed against at least one bacterium.

As used herein, "bactericide activity" refers to killing bacteria, in particular of at least one type of bacteria.

As used herein, "bacteriostatic activity" herein refers to stopping bacteria from reproducing, while not necessarily killing them, in other words bacteriostatic activity herein refers to inhibiting the growth of bacteria. Accordingly, bacteriostatic activity may be expressed, for example, in % of growth inhibition of at least one bacterium.

The "growth inhibition of at least one bacterium" in context of the present invention, may be more than 70%, for example, more than 75, more than 80%, typically, more than 82, 84, 86, 88, 90, 91, 92, 93, 94, 95, 96, 97, 98%.

Accordingly, in one embodiment, the antimicrobial peptide used in the context of the invention has more than 70% growth inhibition of at least one bacterium, more particularly, more than 75%, more than 80%, typically, more than 82, 84, 86, 88, 90, 91, 92, 93, 94, 95, 96, 97, 98% growth inhibition of at least one bacterium.

The "at least one bacterium" herein refers to bacteria of at least 1, 2, 3, 4, 5, 6, 7, 8, 9, 10 or more species of bacteria.

In one embodiment, the at least one bacterium is a ESKAPE pathogen.

The "ESKAPE pathogens" are the leading cause of nosocomial infections throughout the world and are described in, for example, Biomed Res Int. 2016; 2016: 2475067. In one embodiment, the term "ESKAPE pathogens" refers to a bacterium selected from the group constituted of *Enterococcus faecium, Staphylococcus aureus, Klebsiella pneumoniae, Acinetobacter baumannii, Pseudomonas aeruginosa,* and *Enterobacter* species.

In one embodiment, the at least one bacterium is a gram-positive bacterium or gram-negative bacterium, preferably gram-positive bacterium.

In one embodiment, the gram-negative bacterium is a *Pseudomonas aeruginosa, Acinetobacter baumannii, Stenotrophomonas maltophilia, Escherichia coli,Klebsiella pneumoniae, Enterobacter* species *or Legionella* bacterium, preferably, *Escherichia coli or Pseudomonas aeruginosa*

In one embodiment, the gram positive bacterium is a *Staphylococcus, Micrococcus* or *Enterococcus* bacterium.

Bacteria of the *"Staphylococcus"* genus are stationary, non-spore-forming, catalase-positive, oxidase-negative, gram-positive cocci grouped together in grape-like clusters. Observed by Pasteur in 1879 in furuncle pus, staphylococci owe their name to Ogsten (1881) who isolated them in acute chronic abscesses. Bacteria of the "Staphylococcus" genus, such as, for example, *S*. *aureus, S. epidermidis, S. capitis, S. caprae, S. haemolyticus, S. lugdunensis, S. schleiferi, S. simulans* and *S*. *warneri* are the main agents of infections on foreign materials for example in prosthetic joint infections.

Accordingly, in one embodiment the *Staphylococcus* is selected from *S*. *aureus, S. epidermidis, S. capitis, S. caprae, S. haemolyticus, S. lugdunensis, S. schleiferi, S. simulans* and *S*. *warned,* preferably *S*. *aureus and S. epidermidis,* more preferably *S*. *aureus.*

Bacteria of the *"Micrococcus"* genus are generally thought to be a saprotrophic or commensal organism, though it can be an opportunistic pathogen, particularly in hosts with compromised immune systems, such as HIV patients. *Micrococci are* normally present in skin microflora, and the genus is seldom linked to disease. However, in rare cases, death of immunocompromised patients has occurred from pulmonary infections caused by Micrococcus. Micrococci may be involved in other infections, including recurrent bacteremia, septic shock, septic arthritis, endocarditis, meningitis, and cavitating pneumonia in particular in immunosuppressed patients.

In one embodiment the *Micrococcus* is a M. *luteus* bacterium.

Bacteria of the *"Enterococcus"* genus are the cause of important clinical infections such as urinary tract infections, bacteremia, bacterial endocarditis, diverticulitis, and meningitis.

In one embodiment the *Enterococcus* is a vancomycin-resistant *Enterococcus,* such as *E. faecalis* or *E*. *faecium.*

The bacteriostatic activity or % of growth inhibition may be demonstrated, for example, in an antibacterial assay as herein described in the section "methods" herein below. Strains that may be used in such an antibacterial assay may be, for example, *M*. *luteus* or *S*. *aureus.*

The antimicrobial peptide used in the context of the invention is a positively charged peptide.

By "positively charged" is meant herein that the overall peptide charge is positive.

Positively charged amino acids are well-known from the skilled person and include lysine, arginine, histidine and ornithine.

Any positively charged antimicrobial peptide can be used in the context of the invention.

In a particular embodiment, said positively charged antimicrobial peptide is a peptide of following formula (2): wherein 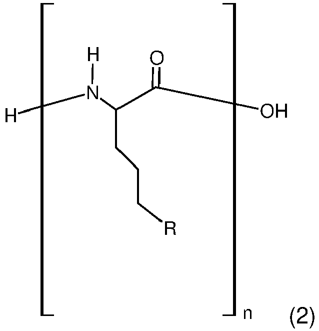
- n is an integer comprised between 2 and 250, in particular between 2 and 200, and
- R is chosen from -NH₂, -CH₂-NH₂ and -NH-C(NH)-NH₂.

The peptide of formula (2) consists of n repetitive units, said repetitive units being identical or different. According to the invention, the repetitive unit of the peptide of formula (2) has the formula -NH-CH(CH₂-CH₂CH₂-R)-C(=O)-. For a given repetitive unit, R is as defined above and may thus be different for each unit.

According to one preferred embodiment, the peptide of formula (2) consists of n repetitive units wherein all the R groups are identical.

According to a further embodiment, the peptide of formula (2) consists of n repetitive units wherein the R groups may be different.

Among the n units, the peptide may comprise i units of formula -NH-CH(CH₂-CH₂-CH₂-NH₂)-C(=O)-, j units of formula -NH-CH(CH₂-CH₂-CH₂-CH₂-NH₂)-C(=O)-, and k units of formula -NH-CH(CH₂-CH₂-CH₂-NH-C(NH)-NH₂)-C(=O)-, wherein each i, j, and k is comprised between 0 and n, and wherein i+j+k=n, with a random distribution of the units or with a distribution as blocks.

In one embodiment, the "n" of the n repetitive units having the formula (2) is an integer comprised between 11 and 200, when R is chosen from -NH₂, -CH₂-NH₂ and - NH-C(NH)-NH₂.

In a further embodiment, n is an integer comprised between 11 and 99, for example, n is an integer comprised between 11 and 95, 15 and 95, 15 and 90, 15 and 85, 15 and 80, 15 and 75, 20 and 95, 20 and 90, 20 and 85, 20 and 80, 20 and 75, 25 and 95, 25 and 90, 25 and 85, 25 and 80, 25 and 75, 28 and 74, 28 and 72, 30 and 70, such as 30, 50 and 70, when R is chosen from -NH₂, -CH₂-NH₂ and -NH-C(NH)-NH₂, preferably, when R is chosen from -CH₂-NH₂ and -NH-C(NH)-NH₂, more preferably, when R is -NH-C(NH)-NH₂.

In a further embodiment, n is an integer comprised between 11 and 49, for example, n is an integer comprised between 11 and 45, 15 and 45, 20 and 40, 21 and 39, 22 and 38, 23 and 37, 24 and 36, 25 and 35, 26 and 34, 27 and 33, 28 and 32, 29 and 31, when R is chosen from -NH₂, -CH₂-NH₂ and -NH-C(NH)-NH₂, preferably, when R is chosen from -CH₂-NH₂ and -NH-C(NH)-NH₂, more preferably, when R is -NH-C(NH)-NH₂.

In one particular embodiment, n is an integer selected from the group consisting of 11, 15, 20, 21, 22, 23, 24, 25, 26, 27, 28, 29, 30, 31, 32, 33, 34, 35, 36, 37, 38, 39, 40, 45, 47, 50, 52, 54, 56, 58, 60, 62, 64, 66, 68, 70, 72, 74, 76, 78, 80, 82, 84, 86, 88, 90, 92, 94, 96, 98, preferably, n is 30, 50 or 70, when R is chosen from -NH₂, -CH₂-NH₂ and -NH-C(NH)-NH₂, preferably, when R is chosen from -CH₂-NH₂ and -NH-C(NH)-NH₂, more preferably, when R is -NH-C(NH)-NH₂.

In one particular embodiment, n is an integer selected from the group consisting of 11, 15, 20, 21, 22, 23, 24, 25, 26, 27, 28, 29, 30, 31, 32, 33, 34, 35, 36, 37, 38, 39, 40, 45 and 49, preferably, n is 30, when R is chosen from -NH₂, -CH₂-NH₂ and -NH-C(NH)-NH₂, preferably, when R is chosen from -CH₂-NH₂ and -NH-C(NH)-NH₂, more preferably, when R is -NH-C(NH)-NH₂.

The "repetitive unit" of formula (2) can also be called "structural unit" and herein refers to an amino acid or amino acid residue, wherein said amino acid is ornithine when R is -NH₂, lysine when R is -CH₂-NH₂ or arginine, when R is -H-C(NH)-NH₂. Accordingly, "n repetitive units of formula (2)" may also be referred to as "n amino acid residues of formula (2)", more precisely as n ornithine residues when R is -NH₂, n lysine residues when R is -CH₂-NH₂ or n arginine residues when R is -H-C(NH)-NH₂.

According to the above, in some embodiments, "n repetitive units having the formula (2)" may be referred to as "polyornithine having n ornithine residues" when R is - NH₂, "polylysine having n lysine residues" when R is -CH₂-NH₂ or "polyarginine having n arginine residues" when R is -H-C(NH)-NH₂.

"Ornithine" is a non proteinogenic amino acid that plays a role in the urea cycle. Polyornithine refers to a polymer of the structural unit ornithine. Polyornithine refers to poly-L-, poly-D- or poly-LD-ornithine. In context of the present invention, polyornithine refers in particular to poly-L-ornithine (PLO).

"Arginine" and "Lysine" are α-amino acids that are used in the biosynthesis of proteins. Polyarginine and -lysine refer to a polymer of the structural unit arginine or lysine, respectively. Polyarginine or -lysine refer to poly-L-, poly-D- or poly-LD-arginine or - lysine. In context of the present invention, polyarginine or polylysine refer, in particular, to poly-L-arginine (PAR) and poly-L-lysine (PLL), respectively.

In a particular embodiment, said positively charged antimicrobial peptide is selected from the group consisting of polyarginine, polyornithine and polylysine.

"Poly-L-ornithine", "poly-L-lysine" and "poly-L-arginine" are positively charged synthetic polymers and are produced in the form of a salt with a counterion. The counter ion may be selected from, but is not limited to, hydrochloride, hydrobromide or trifluoracetate.

In one example, polyarginine is poly-L-arginine hydrochloride with CAS#26982-20-7.

In one example, polyornithine is poly-L-ornithine hydrobromide with CAS#27378-49-0 or poly-L-ornithine hydrochloride with CAS#26982-21-8.

In one example, polylysine is poly-L-lysine trifluoracetate, poly-L-lysine hydrobromide with CAS#25988-63-0 or poly-L-lysine hydrochloride with CAS#26124-78-7.

Poly-L-ornithine, poly-L-lysine and poly-L-arginine having a defined number of amino acid residues may be obtained commercially, for example, via Alamanda Polymers, USA.

In one example, poly-L-arginine (PAR) such as PAR10 (10 arginine (R), Mw = 2.1 kDa, PDI = 1); PAR30 (30 R, Mw = 6.4 kDa, PDI, = 1.01), PAR50 (50 arginine (R), Mw = 9.6 kDa, PDI= 1.03); PAR70 (70 arginine (R), Mw = 13.4 kDa, PDI, = 1.01), PAR100 (100 R, Mw = 20.6 kDa, PDI = 1.05), and PAR200 (200 R, Mw = 40.8 kDa, PDI = 1.06) were purchased from Alamanda Polymers, USA.

In another example, poly-L-ornithine (PLO) such as PLO30 (30 R, Mw = 5.9 kDa, PDI = 1.03), PLO100 (100 R, Mw = 18.5 kDa, PDI = 1.03), and PLO250 (250 R, Mw = 44.7 kDa, PDI = 1.02) were purchased from Alamanda Polymers, USA.

In a further example poly-L-lysine (PLL) such as PLL10 (10 R, Mw = 1.6 kDa), PLL30 (30 R, Mw = 5.4 kDa, PDI = 1.02), PLL100 (100 R, Mw = 17.3 kDa, PDI = 1.07), PLL250 (250 R, Mw = 39.5 kDa, PDI = 1.08) was purchased from Alamanda Polymers, USA.

Methods to obtain polypeptides having n repetitive units such as polyarginine, polylysine, or polyornithine with for example n=30 are known to the skilled in the art and include ring-opening polymerization of alpha-amino acid N-carboxyanhydrides (NCAs) followed by purification. Typically, the polypeptides are purified after polymerization by precipitation in water or, for example, in an organic non-solvent and, after amino acid side chain deprotection, by dialysis. All water-soluble polymers are finally lyophilized.

In a particularly preferred embodiment, said positively charged antimicrobial peptide is polyarginine.

In a more particularly preferred embodiment, said polyarginine is of the following formula (1) 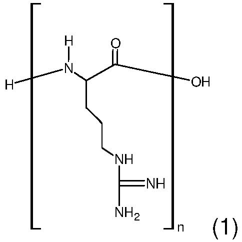 wherein n is an integer comprised between 2 and 100.

In a further embodiment, n, in formula (1), is an integer comprised between 11 and 99, for example, n is an integer comprised between 11 and 95, 15 and 95, 15 and 90, 15 and 85, 15 and 80, 15 and 75, 20 and 95, 20 and 90, 20 and 85, 20 and 80, 20 and 75, 25 and 95, 25 and 90, 25 and 85, 25 and 80, 25 and 75, 28 and 74, 28 and 72, 30 and 70, such as 30, 50 and 70.

In a further embodiment, n, in formula (1) is an integer comprised between 11 and 49, for example, n is an integer comprised between 11 and 45, 15 and 45, 20 and 40, 21 and 39, 22 and 38, 23 and 37, 24 and 36, 25 and 35, 26 and 34, 27 and 33, 28 and 32, 29 and 31.

In one particular embodiment, n, in formula (1) is an integer selected from the group consisting of 11, 15, 20, 21, 22, 23, 24, 25, 26, 27, 28, 29, 30, 31, 32, 33, 34, 35, 36, 37, 38, 39, 40, 45, 47, 50, 52, 54, 56, 58, 60, 62, 64, 66, 68, 70, 72, 74, 76, 78, 80, 82, 84, 86, 88, 90, 92, 94, 96, 98, preferably, n is 30, 50 or 70.

In one particular embodiment, n, in formula (1), is an integer selected from the group consisting of 11, 15, 20, 21, 22, 23, 24, 25, 26, 27, 28, 29, 30, 31, 32, 33, 34, 35, 36, 37, 38, 39, 40, 45 and 49, preferably, n is 30.

In a particularly preferred embodiment, said polyarginine is of the following formula (1) 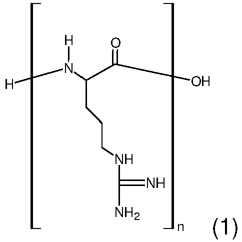 wherein n is 30.

In a particular embodiment, said hydrogel is loaded with a positively charged antimicrobial peptide of formula (2) as defined above, wherein n is n₁, an integer comprised between 2 and 100, and with at least a positively charged antimicrobial peptide of formula (2) as defined above, wherein n is n₂, an integer comprised between 2 and 100 but different from n₁.

In other words, in a particular embodiment, said hydrogel is loaded with at least two positively charged antimicrobial peptides of formula (2), said peptides being of different sizes.

In a particular embodiment, said hydrogel is loaded with polyarginine of the following formula (1) 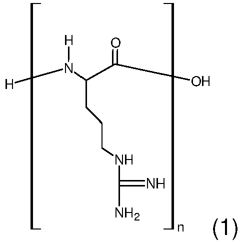 wherein n is n₁, an integer comprised between 2 and 250, in particular between 2 and 200, and with polyarginine of the following formula (1) 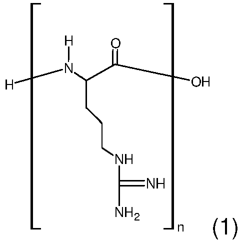 wherein n is n₂, an integer comprised between 2 and 250, in particular between 2 and 200, wherein n₂ is different from n₁.

In a more particular embodiment, said hydrogel is loaded with polyarginines of formula (1) of different sizes.

In another embodiment, the antimicrobial peptide is selected from catestatin, cateslytin, polyornithine, polylysine and their D- or L-isomers, nisin, defensing, mellitin and magainin.

In a particular embodiment, the hydrogel of the invention is loaded with different positively charged antimicrobial peptides as defined above, for example with a mixture of PAR10 and PAR200 or with a mixture of PAR30 and PAR200.

### Additional compounds

The hydrogel of the invention may further comprise additional compounds. In particular, the hydrogel of the invention may further comprise a pharmaceutical active drug.

In the context of the present invention, the term "pharmaceutical active drug" refers to compounds or entities which alter, inhibit, activate or otherwise affect biological events. For example, the drug includes, but is not limited to, anti-cancer substances, anti-inflammatory agents, immunosuppressants, modulators of cell-extracellular matrix interaction including cell growth inhibitors, anticoagulants, antrithrombotic agents, enzyme inhibitors, analgetic, antiproliferative agents, antimycotic substances, cytostatic substances, growth factors, hormones, steroids, non-steroidal substances, and antihistamines. Examples of indication groups are, without being limited thereto analgetic, antiproliferativ, antithrombotic, anti-inflammatory, antimycotic, antibiotic, cytostatic, immunosuppressive substances as well as growth factors, hormones, glucocorticoids, steroids, non-steroidal substances, genetically or metabolically active substances for silencing and transfection, antibodies, peptides, receptors, ligands, and any pharmaceutical acceptable derivative thereof. Specific examples for above groups are paclitaxel, estradiol, sirolimus, erythromycin, clarithromycin, doxorubicin, irinotecan, gentamycin, dicloxacillin, quinine, morphin, heparin, naproxen, prednisone, dexamethasone, cytokines, IL-4, IL-10, VEGF, fungizone, catestatin or cateslytin.

### Method of preparation

The present invention also concerns a method for preparing a hydrogel as defined above, wherein said method comprises the following steps:
(a) mixing, in basic conditions, hyaluronic acid (HA) or a derivative thereof, as defined in the section *"Hyaluronic acid hydrogel*" above, with a cross-linking agent which cross-links HA at the level of its hydroxyl moieties while the carboxyl moieties of HA or derivative thereof remain free and said HA or derivative thereof remains negatively charged, as defined in the section *"Hyaluronic acid hydrogel*" above,
(b) depositing the mixture on a support and incubating it for 48 h to 72 h at room temperature to obtain a hydrogel,
(c) recovering the hydrogel formed at step (b),
(d) incubating said hydrogel in an aqueous buffer in conditions enabling the withdrawal of the cross-linking agent residues and the hydrogel to swell,
(e) loading the hydrogel obtained at step (d) with at least one positively charged antimicrobial peptide, as defined in the section *"Positively charged antimicrobial peptide"* above, without said peptide being covalently linked to the hydrogel, and
(f) recovering the loaded hydrogel obtained at step (e).

### Mixing step (a)

The mixing step (a) of the method of the invention consists in mixing, in basic conditions, hyaluronic acid (HA) or a derivative thereof, as defined in the section *"Hyaluronic acid hydrogel*" above, with a cross-linking agent which cross-links HA at the level of its hydroxyl moieties while the carboxyl moieties of HA or derivative thereof remain free and said HA or derivative thereof remains negatively charged, as defined in the section *"Hyaluronic acid hydrogel" above.*

In a particular embodiment, HA is a hyaluronic acid having a molecular weight of between 800 and 850 kDa, in particular having a molecular weight of 823 kDa.

In a particular embodiment, the mixture of step (a) comprises from 1 to 10% (w/v) of HA or derivative thereof as defined above, more preferably from 2 to 3% (w/v) of HA or derivative thereof as defined above, most preferably 2.5% (w/v) of HA or derivative thereof as defined above.

In a particular embodiment, the cross-linking agent is butanediol diglycidyl ether (BDDE).

In a particular embodiment, the mixture of step (a) comprises at least 10% (v/v) of cross-linking agent as defined above, in particular of BDDE, more preferably at least 20% (v/v) of cross-linking agent, in particular of BDDE. In a particular embodiment, the mixture of step (a) comprises from 10% to 30% (v/v) of cross-linking agent as defined above, in particular of BDDE.

In a particular embodiment, the mixture of step (a) comprises from 2 to 3 % (w/v) of HA or derivative thereof, and at least 10% (v/v) of cross-linking agent, in particular of BDDE, in particular at least 20% (v/v) of cross-linking agent, in particular of BDDE.

By "basic conditions" is meant herein, reactive conditions wherein the pH is above 7. Typically, HA and the cross-linking agent are mixed in NaOH solution, in particular in 0.1 M to 0.3 M NaOH solution, more particularly in 0.25 M NaOH solution.

### Deposition step (b)

The deposition step (b) of the method of the invention consists in depositing the mixture obtained at step (a) on a support and incubating it for 48 h to 72 h at room temperature to obtain a hydrogel, as defined above.

Any suitable suitable support can used such as a Petri dish, a glass slide, a well plate, parafilm, medical compresses, meshes or medical prostheses (polymeric, metallic).

Said support may be in any suitable material such as polyethylene terephthalate (PET), polytetrafluoroethylene (PTFE), polyprolylene, acrylonitrile butadiene styrene (ABS), glass, Teflon or metal. In a particular embodiment, said support is in a material capable to support a high pH.

Said support can be pre-treated before deposition of the mixture. For example, said support may be washed, for example washed in Hellmanex^{®} (an anionic detergent) solution and/or in HCl solution and/or in 70% alcohol solution and/or in acetone, and/or treated for improving adhesion for example by deposition of polyethyleneimine (PEl).

The mixture can be deposited on the support by any technique well-known from the skilled person such as drop-deposition, pouring, pipetting, extrusion, spin-coating, dipping or 3D printing.

The mixture, once deposited on the support, is incubated for 48 h to 72 h, preferably for 72 h, at room temperature, to obtain a hydrogel as defined above.

### Recovering step (c)

The hydrogel formed at step (b) is then recovered.

Recovering step (c) can be implemented by any technique well-known from the skilled person, for example by cutting pieces of the hydrogel typically using a circle cutter, scalpel, precision cutting instruments (physical or laser based) and specific press-cutter according to size need.

### Incubation step (d)

The incubation step (d) of the method of the invention consists in incubating said hydrogel in an aqueous buffer in conditions enabling the withdrawal of cross-linking agent residues and the hydrogel to swell.

By "aqueous buffer" is meant herein an aqueous solution consisting of a mixture of weak acid and its conjugate base, or vice versa. Examples of aqueous buffer include Tris/NaCl buffers (for example Tris 10 mM, NaCl 0.15 M, pH 7.4 buffer), PBS or HEPES.

As used herein, aqueous buffer further encompasses a solution consisting of water, such as distilled water.

By "withdrawal of cross-linking agent residues" is meant herein that cross-linking agent molecules not involved in bonding are eliminated from the hydrogel, such that there is preferably no more than 0.01 % free cross-linking agent molecules in the hydrogel after the incubation step.

By "swelling of hydrogel" is meant herein that the hydrogel capture water from the aqueous buffer in which it is incubated, preferably until a level at which the hydrogel increases at least of 1.5 fold its size.

Incubation step is typically carried out for 1-5 min to 1 h. The incubation step may in particular include several incubations in different or same buffers, typically a first incubation during 1-5 min and a second incubation during 1 h.

The hydrogel can typically be stored at 4°C after the incubation step before implementing the loading step.

### Loading step (e)

The loading step (e) of the method of the invention consists in loading the hydrogel obtained at step (d) with at least one positively charged antimicrobial peptide, as defined in the section *"Positively charged antimicrobial peptide"* above.

In a particular embodiment, the positively charged antimicrobial peptide is polyarginine as defined in the section *"Positively charged antimicrobial peptide"* above, in particular PAR30.

In a particular embodiment, said positively charged antimicrobial peptide is loaded at step (e) at a concentration of 0.05 to 5 mg/ml, more particularly of 0.05 to 1 mg/ml.

Said loading is preferably carried out at room temperature.

The loading of the positively charged antimicrobial peptide can be carried out for 2 h to 48 h, in particular for 3 h to 24 h, preferably for 24 h.

The loading step (e) can be following by a washing step (e'), wherein the loaded hydrogel is washed in aqueous buffer as defined above, in particular in Tris Nacl buffer.

The loaded hydrogel prepared by the method of the invention may further comprise additional compounds as defined in the section *"Additional compounds"* above. Such additional compounds can be added during the formation of the hydrogel or during the loading of the hydrogel. In particular, said additional compounds may be mixed with HA before the addition of the cross-linking agent, may be mixed with the mixture of HA and the cross-linking agent, or may be loaded after the cross-linking reaction before or after the loading with the antimicrobial peptide.

The present invention further concerns a hydrogel likely to be obtained by the method of preparation as defined above.

As will be clearly apparent to the skilled person from the method of preparation disclosed above, the hydrogel likely to be obtained by the method of preparation as defined above has a high cross-linking level.

### Medical device

The present invention further concerns a medical device comprising a hydrogel as defined above.

By "medical device" is meant herein items such as catheters, stents, endotracheal tubes, hypotubes, filters such as those for embolic protection, surgical instruments and the like. Any device that is typically coated in the medical arts can be used in the present invention. It is further in the scope of the invention, wherein the term refers to any material, natural or artificial that is inserted into a mammal. Particular medical devices especially suited for application of the hydrogel of this invention include, but are not limited to, peripherally insertable central venous catheters, dialysis catheters, long term tunneled central venous catheters, long term non-tunneled central venous catheters, peripheral venous catheters, short-term central venous catheters, arterial catheters, pulmonary artery Swan-Ganz catheters, urinary catheters, artificial urinary sphincters, long term urinary devices, urinary dilators, urinary stents, other urinary devices, tissue bonding urinary devices, penile prostheses, vascular grafts, vascular catheter ports, vascular dilators, extravascular dilators, vascular stents, extravascular stents, wound drain tubes, hydrocephalus shunts, ventricular catheters, peritoneal catheters, pacemaker systems, small or temporary joint replacements, heart valves, cardiac assist devices and the like and bone prosthesis, joint prosthesis and dental prosthesis.

The term "medical device" as used herein further encompasses wound dressing and mesh prosthesis.

The term "wound dressing" refers hereinafter to any pharmaceutically acceptable wound covering, such as:
a) films, including those semipermeable or a semi-occlusive nature such as polyurethane copolymers, acrylamides, acrylates, paraffin, polysaccharides, cellophane and lanolin;
b) hydrocolloids including carboxymethylcellulose, protein constituents of gelatin, pectin, and complex polysaccharides including acacia gum, guar gum and karaya, which may be utilized in the form of a flexible foam or, in the alternative, formulated in polyurethane or, in a further alternative, formulated as an adhesive mass such as polyisobutylene;
c) impregnates including pine mesh gauze, paraffin and lanolin-coated gauze, polyethylene glycol-coated gauze, knitted viscose, rayon, and polyester; and
d) cellulose-like polysaccharides such as alginates, including calcium alginate, which may be formulated as non-woven composites of fibers or spun into woven composites.

In a particular embodiment, said wound dressing comprises as carrier material nonwoven or knit fabrics, knitted or woven fabrics made on natural or synthetic fibers.

By "mesh prosthesis" is meant herein a loosely woven sheet which is used as either a permanent or temporary support for organs and other tissues during surgery. Mesh prosthesis can typically be polypropylene mesh, polyethylene terephthalate mesh, polytetrafluorethylene mesh or polyvinylidene fluoride mesh.

In a particular embodiment, said medical device is a wound dressing or a mesh prosthesis.

In a particular embodiment, the medical device is coated and/or impregnated with the hydrogel of the invention.

In particular, when the medical device is a wound dressing, the carrier material of the wound dressing is preferably coated or impregnated with the hydrogel on one or more sides.

The hydrogel can be applied to and/or included in the medical device by any method well-known from the skilled person.

Typically, the hydrogel can be applied to and/or included in the medical device, by drop deposition of the un-crosslinked HA-cross-linking agent mixture onto the material or by dipping the absorbent material in the uncrosslinked HA-cross-linking agent mixture, or by coating the hydrogel with a dedicated instrument or extrusion printing.

In a particular embodiment of the invention, it is also provided that the hydrogel according to the present invention is arranged in a package. It is particularly provided that the hydrogel is sterile packaged. In these cases, packages such as containers with screw caps, reclosable tubes, or expendable containers like tubes with safety caps for example, may be used. However, it may also be provided that the hydrogel is arranged in a syringe used as original package. It is particularly provided that the hydrogel in the syringe is sterile. In a particularly preferred embodiment, this hydrogel contained in sterile original package as a ready for use kit is available together with a drug carrier or dressing material, and possibly also further medical aids. It may also be provided that both the hydrogel in the original package and the drug carrier or dressing materials are available in a sterile original package in the kit package.

Any combination of the above embodiments makes part of the invention.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (i.e. "consisting of"). Furthermore the indefinite article "a" or "an" does not exclude a plurality. The mere fact that certain measures are recited in mutually different dependent claims does not indicate that a combination of these measures cannot be used to advantage.

The invention will now be described in more detail with reference to the following examples. All literature and patent documents cited herein are hereby incorporated by reference. While the invention has been illustrated and described in detail in the foregoing description, the examples are to be considered illustrative or exemplary and not restrictive.

### Brief description of the figures

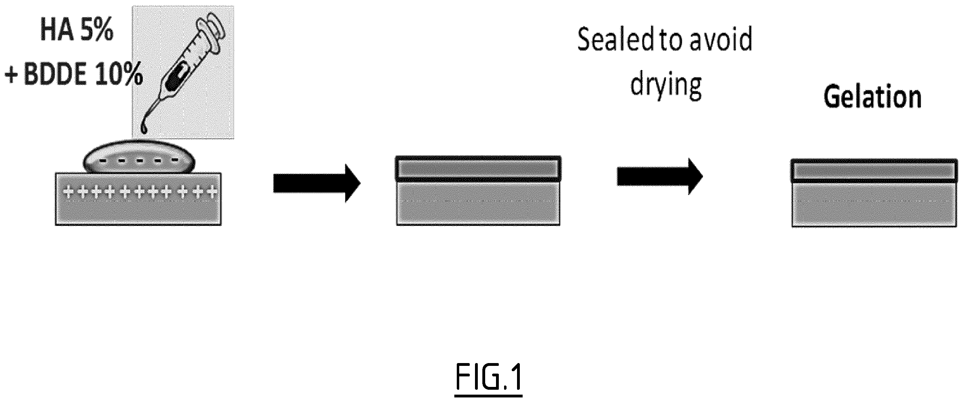

**Figure 1****:** Schematic representation of hydrogel preparation process.

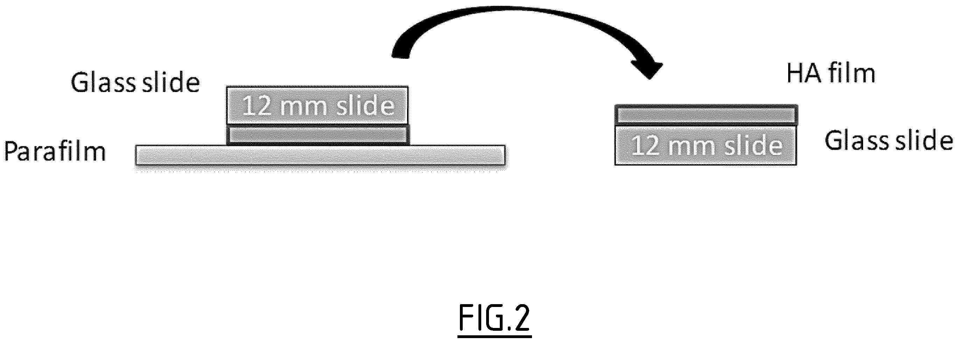

**Figure 2****:** Construction between parafilm and glass slide.

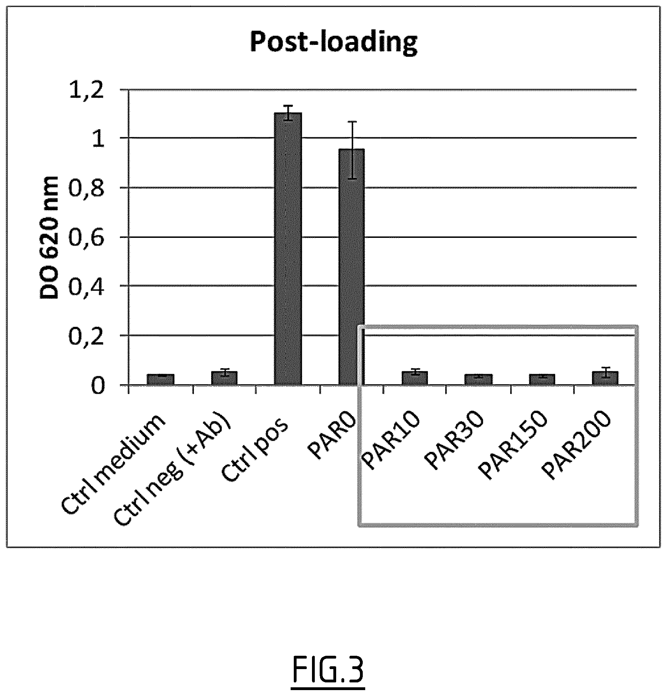

**Figure 3****:** Bacterial growth on PAR pre-loaded and post-loaded HA films. Bacterial growth was evaluated after 24h by optical density (OD) measurement at 620 nm. PARC corresponds to HA film without PAR. HA 2.5% + BDDE 20% films were added with 1 mg/mL PAR after cross-linking (= post-loaded films).

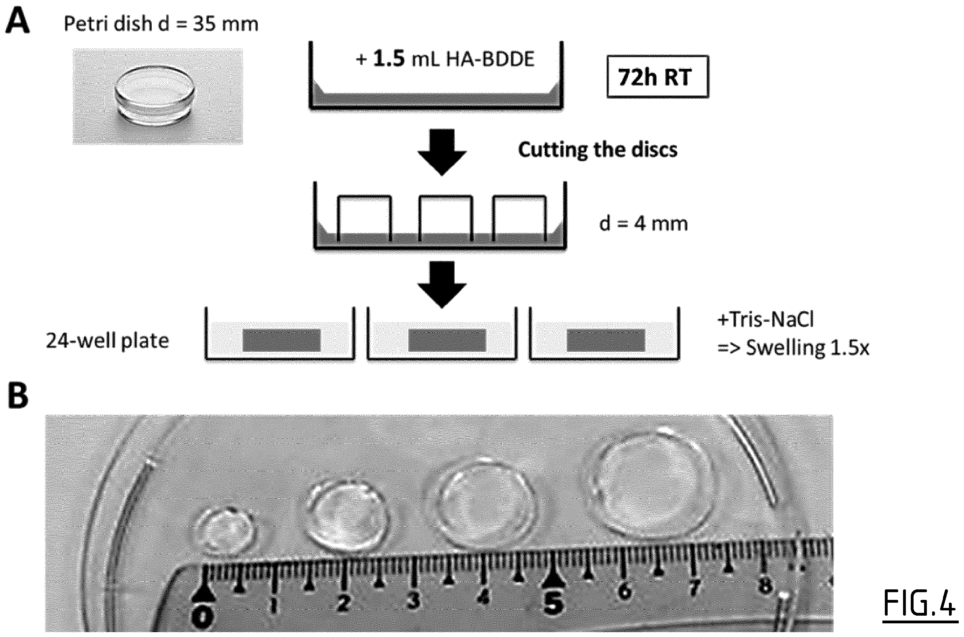

**Figure 4****:** Production of free-standing HA hydrogels. Schematic protocol for hydrogel discs preparation (A) to obtain HA hydrogel discs of different sizes (B).

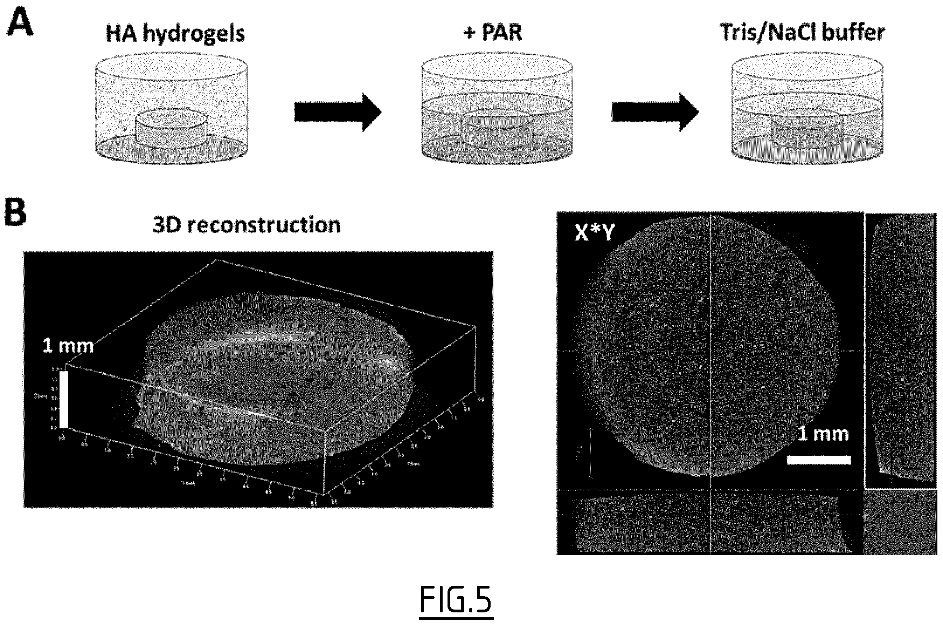

**Figure 5****:** Loading of PAR into HA hydrogel discs. (A) Schematic presentation of PAR-FITC loading. (B) CLSM images of resulting hydrogel discs (loaded with 0.5 mg/mL PAR30-FITC): 3D reconstruction (left) and cross-cuts in Z (right).

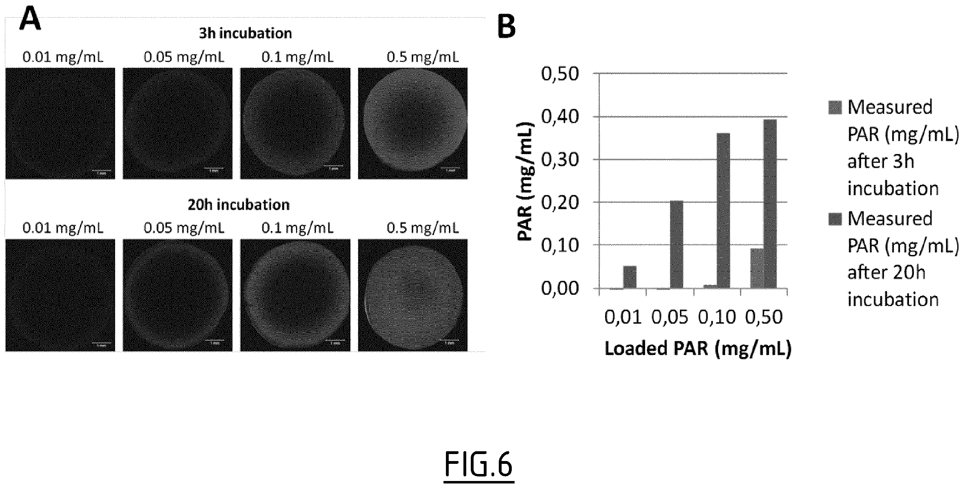

**Figure 6****:** Loading of PAR30-FITC into HA hydrogel discs at different concentrations for 3 h and 24 h. (A) CLSM images (B) Quantification of PAR concentration in the center of the discs.

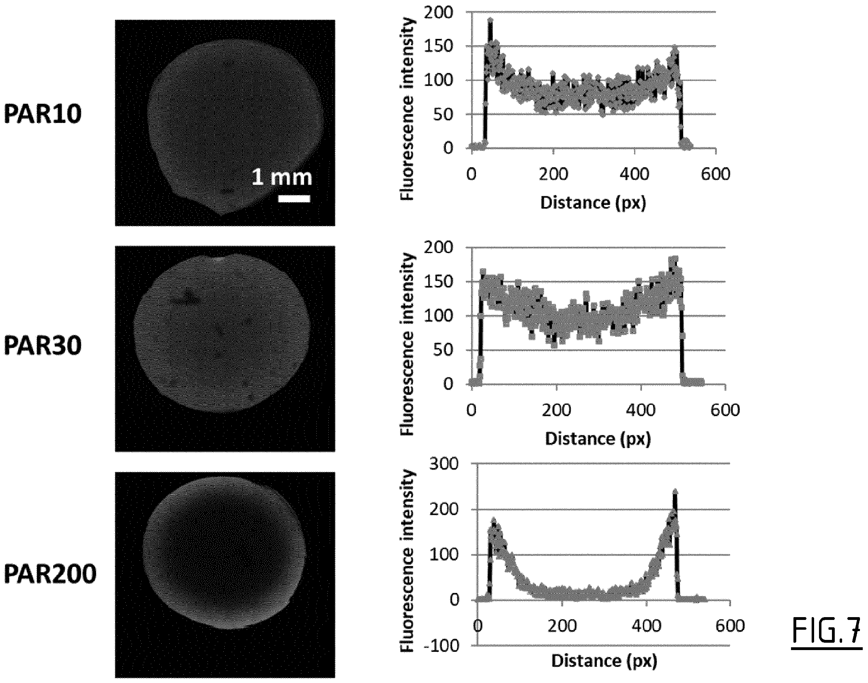

**Figure 7****:** Loading of PARs-FITC into HA hydrogel discs. CSLM images of HA hydrogel discs loaded with 0.5 mg/mL of three PARs labelled with FITC (on the left) and fluorescence profiles of the resulting discs (on the right).

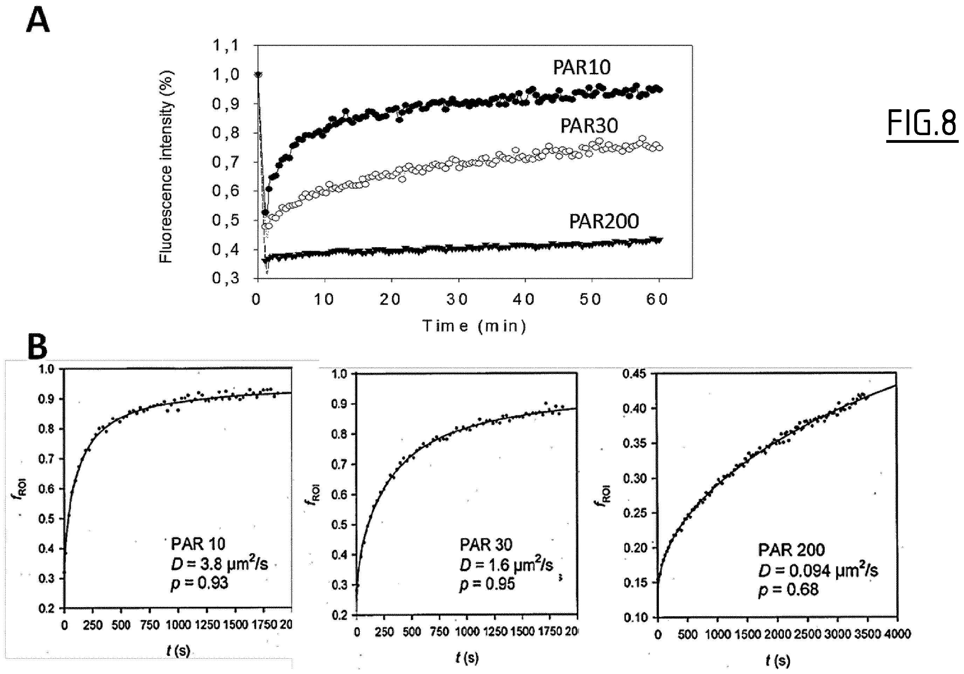

**Figure 8****:** PAR mobility inside HA hydrogels. HA discs were loaded with 0.5 mg/mL of three different FITC-conjugated PARs for 24 h and rinsed. Then, FRAP experiments were conducted. (A) Comparison of the fluorescence recovery of three PARs. (B) Determination of the diffusion coefficients D and percentage of mobile molecules p for three PARs.

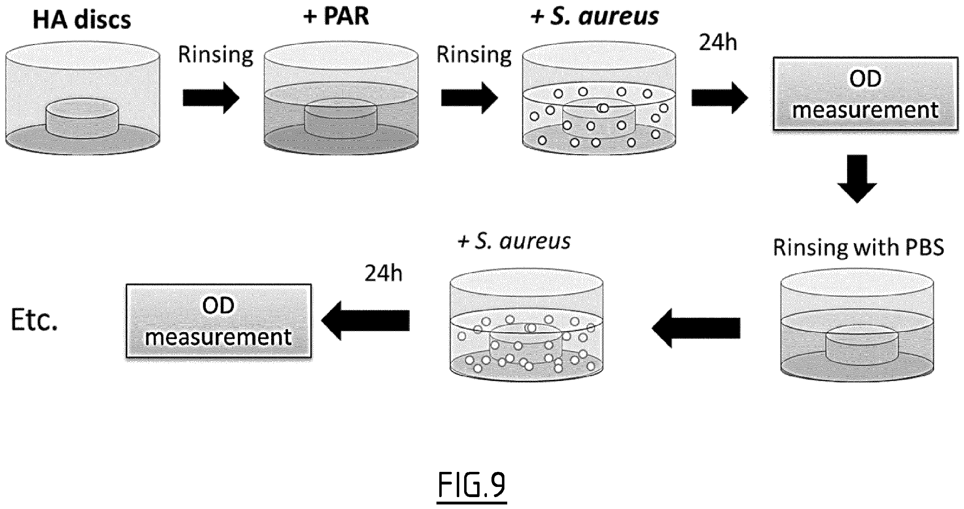

**Figure 9****:** Antibacterial activity of HA hydrogels loaded with PAR10, PAR30, PAR200: repetitive culture. Repetitive culture: after 24h of bacterial culture, the samples were rinsed and seeded with fresh bacteria.

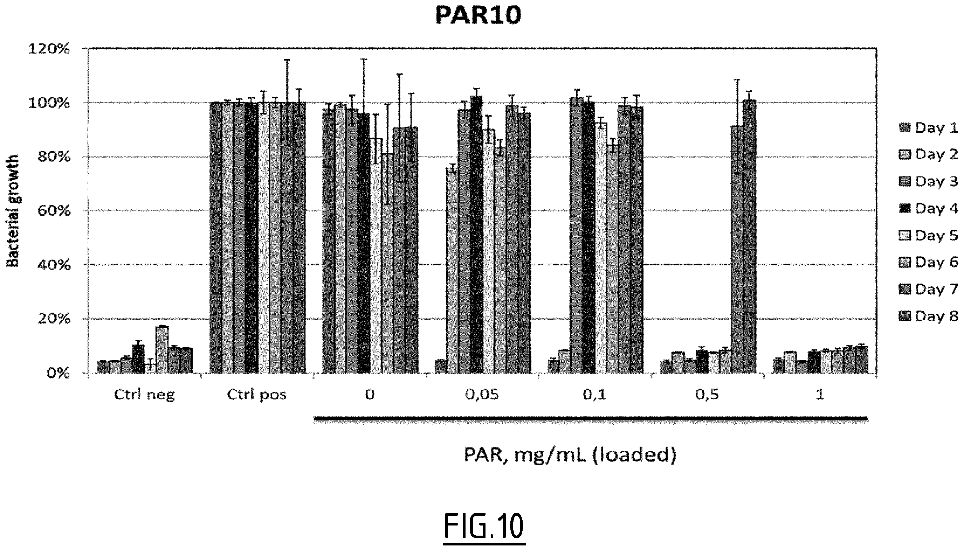

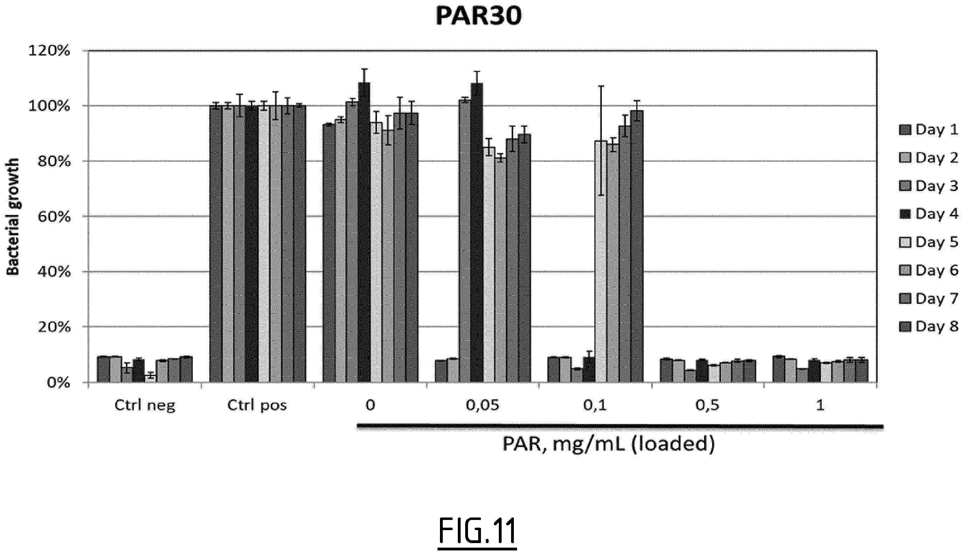

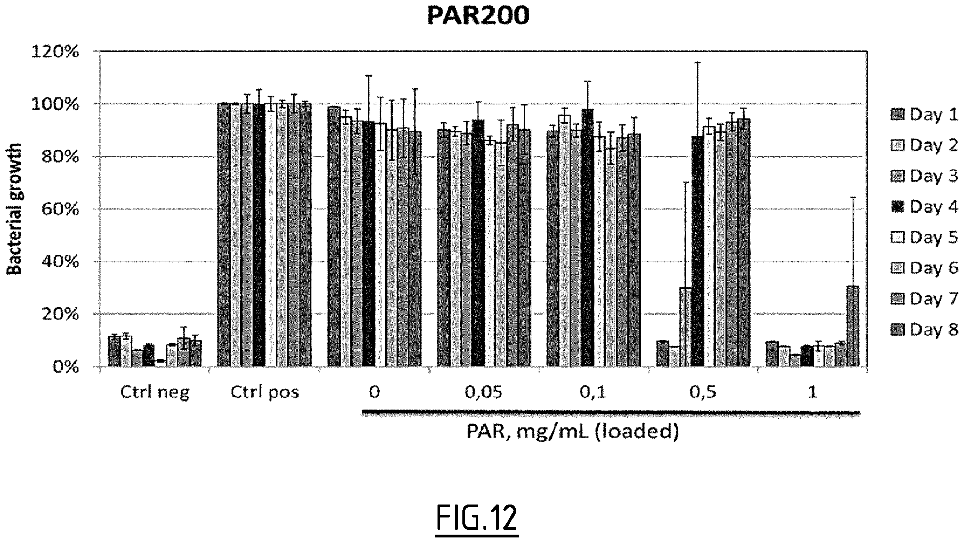

**Figures 10-12****:** Antibacterial activity of HA hydrogels loaded with PAR10, PAR30, PAR200: repetitive culture. After 24h of bacterial culture, the samples were rinsed and seeded with fresh bacteria. The graphs show bacterial growth in presence of hydrogel discs loaded with different concentrations of PAR10 (Figure 10), PAR30 (Figure 11) and PAR200 (Figure 12).

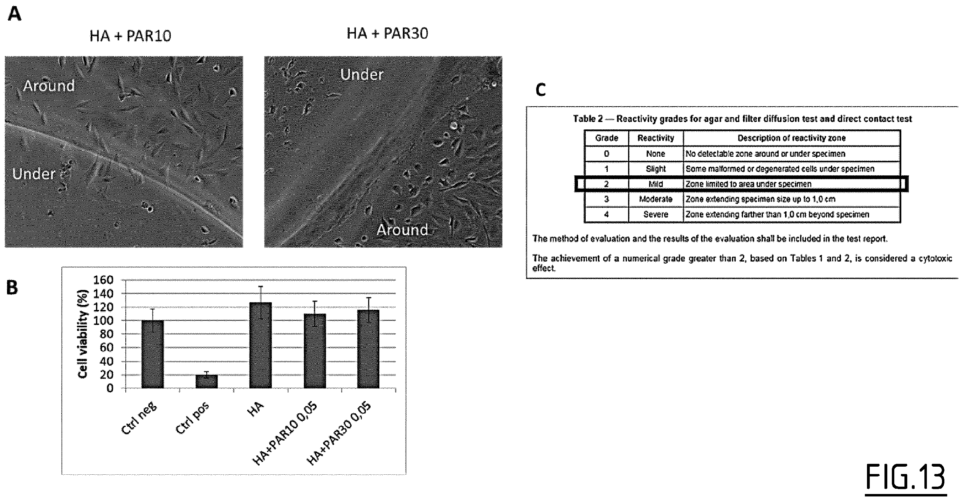

**Figure 13****:** Cytotoxicity assay on PAR10 and PAR30-loaded HA hydrogels. (A). Balb/3T3 cells after 24 h show detachment/deformation under HA+PAR10 and HA+PAR30 discs (loaded at 0.05 mg/mL), while remaining in good health around the discs. (B) MTT test confirmed good cell viability. (C) Such reactivity (grade 2) is considered as mild reactivity without cytotoxic effect, according to ISO 10993.

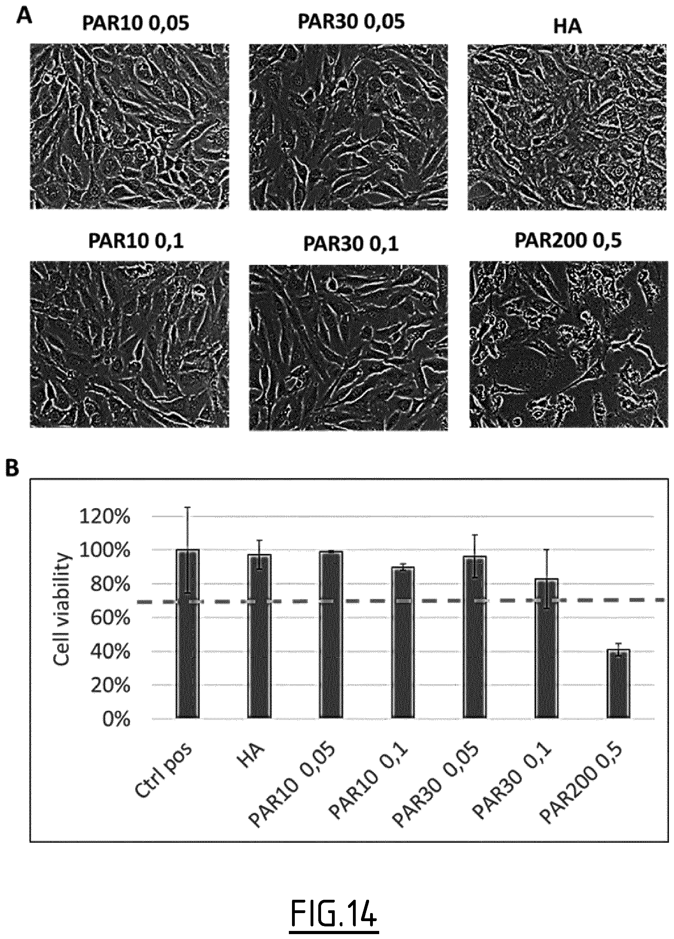

**Figure 14****:** Cell viability evaluation by MTT test. Balb/3T3 cells were seeded in 24-well plate and put in contact with HA hydrogel discs loaded or not with PARs for 24h. HA discs correspond to BDDE-crosslinked HA hydrogel discs without PAR, and HA+PARs correspond to HA hydrogels loaded with different PAR concentrations (mg/mL). (A) Cell images after 24 h of direct *in vitro* cytotoxicity test. (B) Cell viability measured by MTT test.
Dashed line corresponds to 70% viability (cytotoxicity limit).

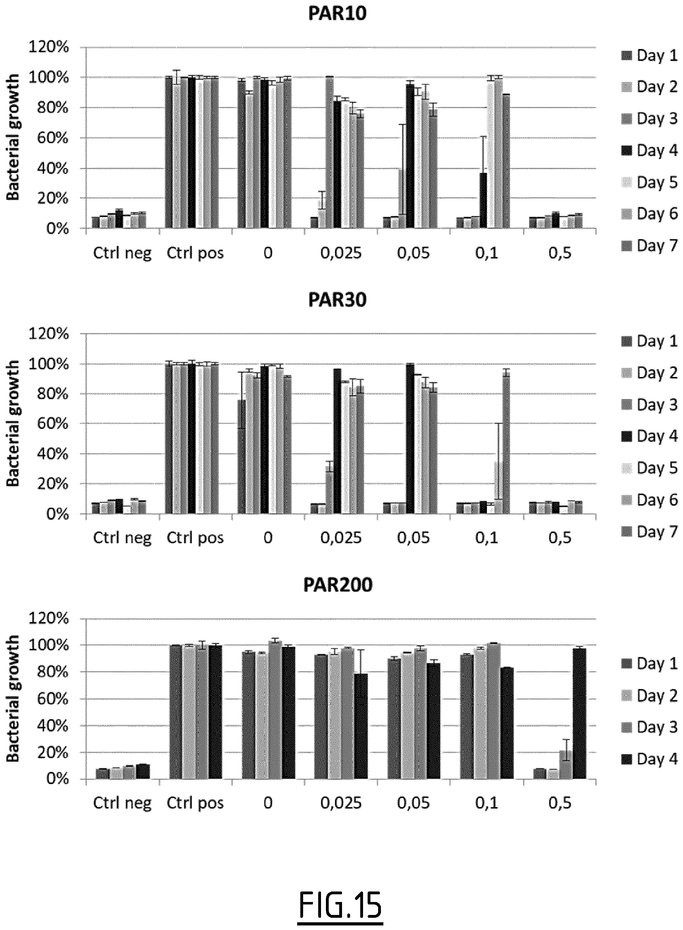

**Figure 15****:** Antibacterial activity of HA hydrogels loaded with PAR10, PAR30, PAR200: repetitive culture. Every 24 h of bacterial culture, the samples were seeded with fresh bacteria. The graphs show bacterial growth in presence of hydrogel discs loaded with PAR10, PAR30 and PAR200 (loading concentration is indicated).

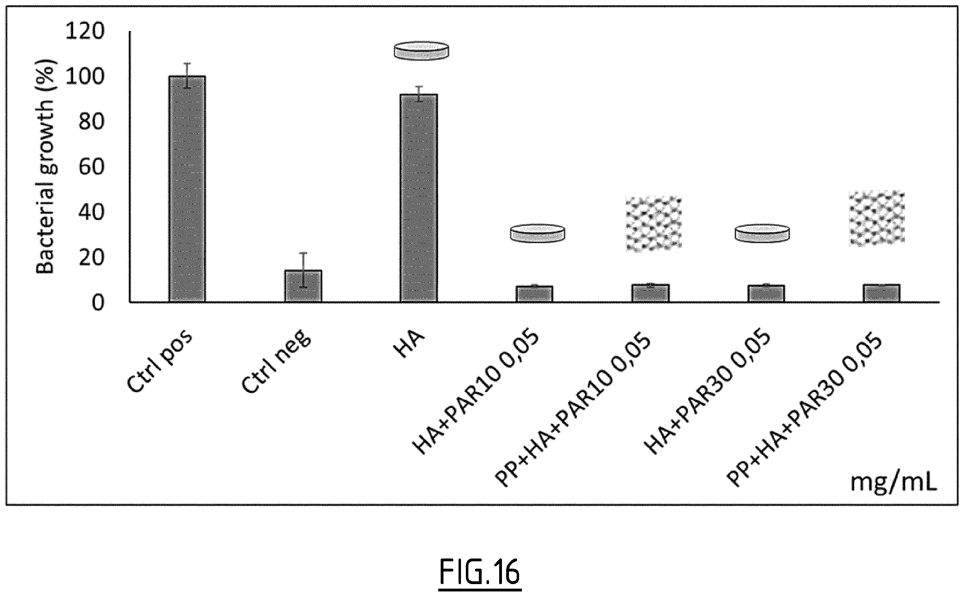

**Figure 16****:** Antibacterial activity of HA hydrogel discs and hydrogel-coated meshes loaded with PAR. The graphs show bacterial growth in presence of hydrogel discs or hydrogel-coated meshes loaded with 0.05 mg/mL of PAR10 or PAR30 (loading concentration is indicated, mg/mL). The bacteria were incubated with materials at 37°C for 6 days, then optical density was measured to evaluate bacterial growth.

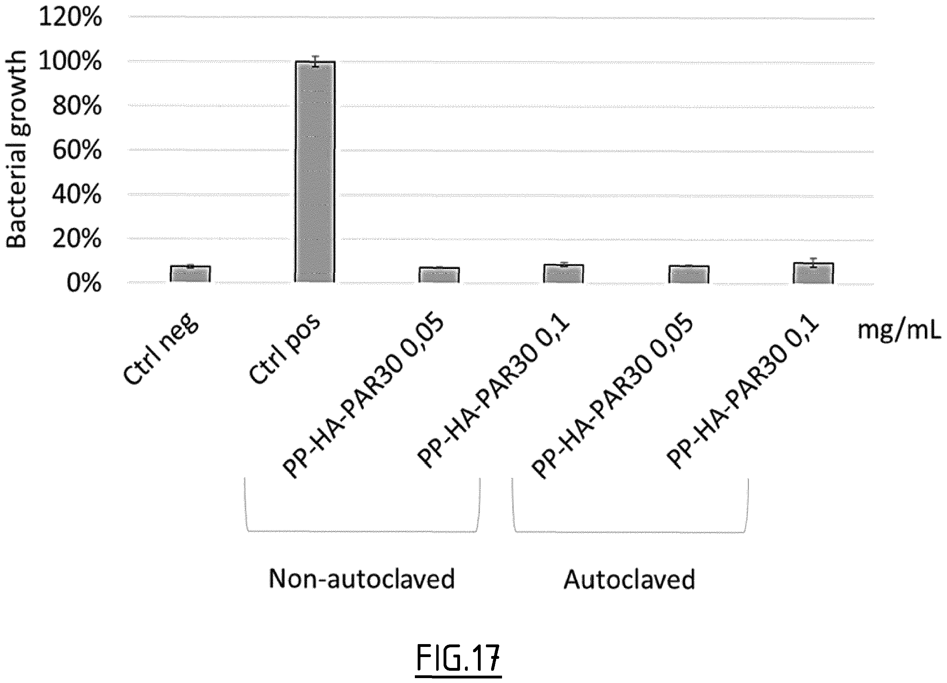

**Figure 17****:** Antibacterial activity of HA hydrogels after autoclaving. The graphs show bacterial growth in presence of hydrogel-coated polypropylene (PP) meshes loaded with 0.05 and 0.1 mg/mL of PAR30 and sterilized by autoclaving, compared to non-autoclaved samples. The bacteria were incubated with materials at 37°C for 24h, then optical density was measured to evaluate bacterial growth.

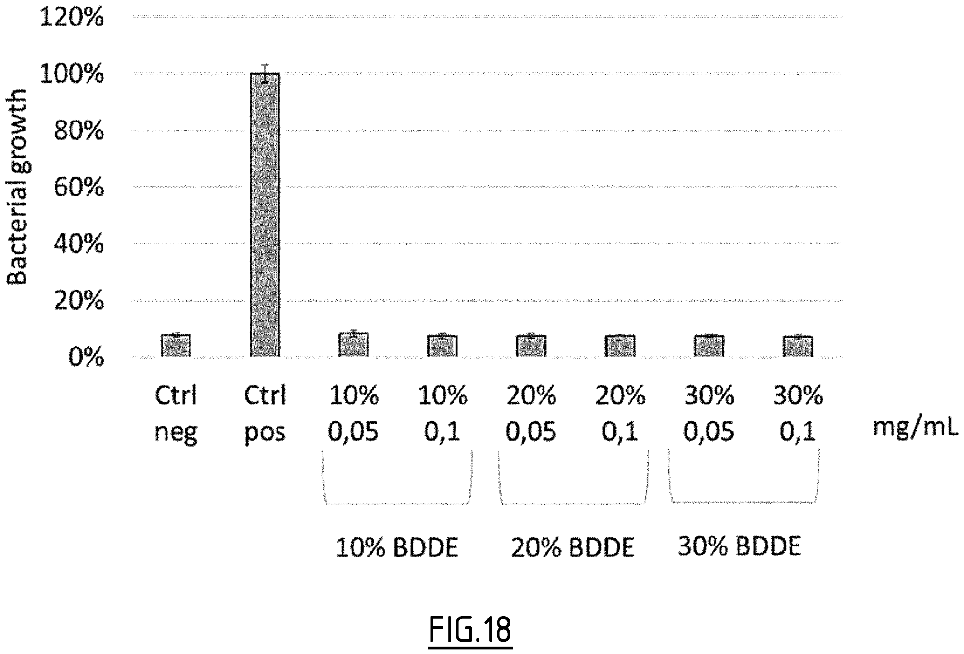

**Figure 18****:** Antibacterial activity of HA hydrogels cross-linked with different % of BDDE. The graphs show bacterial growth in presence of HA hydrogel discs loaded with 0.05 and 0.1 mg/mL of PAR30 and sterilized by autoclaving, compared to non-autoclaved samples. The bacteria were incubated with materials at 37°C for 24 h, then optical density was measured to evaluate bacterial growth.

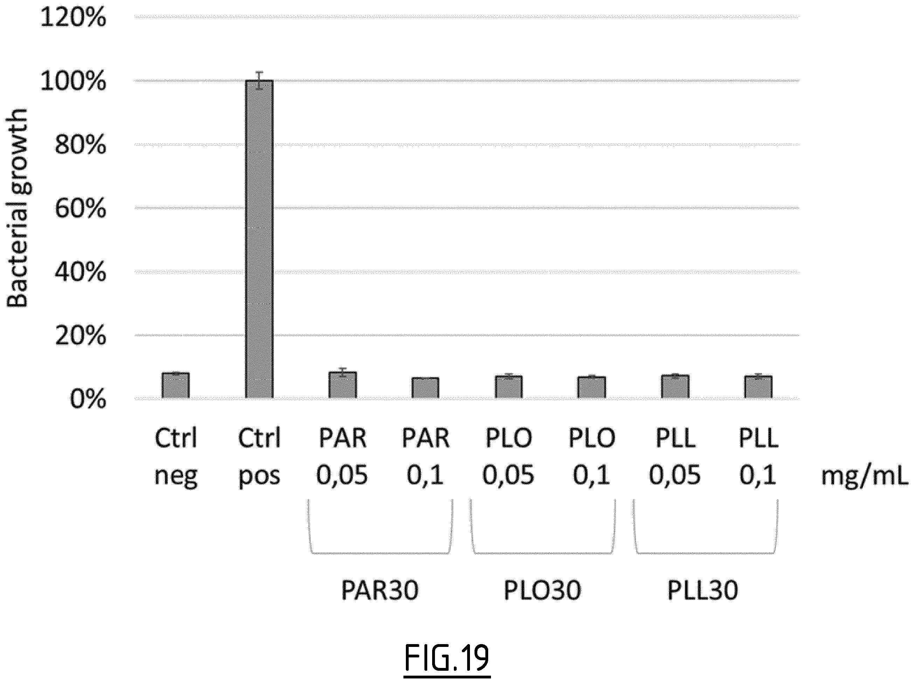

**Figure 19****:** Antibacterial activity of HA hydrogels loaded with positively charged antibacterial polypeptides. The graphs show bacterial growth in presence of HA hydrogel discs loaded with 0.05 and 0.1 mg/mL of polyarginine PAR30, polyornithine PLO30 and polylysine PLL30. The bacteria were incubated with materials at 37°C for 24h, then optical density was measured to evaluate bacterial growth.
**Figures 20-24****:** Release of PARs in NaCl and in culture media. HA discs were incubated with 0.5 mg.mL⁻¹ of three different FITC-conjugated PAR for 24h. The discs were rinsed, then incubated for 72h in NaCl 1M, MH or DMEM.
Figure 20: Confocal microscopy observations, percentage of PAR remaining after 72h is indicated.
Figure 21: Quantification by spectrofluorimetry of PAR release after 72h in NaCl 1M. The graphs represent averages from 3 independent experiments, and error bars represent standard deviations.
Figure 22: Confocal microscopy images of the discs before and after incubation with MH and DMEM.
Figure 23: Percentage of released PARs in MH, where 100% represent fluorescence intensity of the discs in Tris/NaCl.
Figure 24: Percentage of released PARs in DMEM, where 100% represent fluorescence intensity of the discs in Tris/NaCl.

### Examples

### EXAMPLE 1: HA cross-linking and hydrogel deposition

As a first step of HA hydrogel development, the inventors evaluated several substrates and deposition techniques. The goal was to obtain 10 - 100 µm thick and homogeneous HA layers.

To cross-link the films, the inventors chose butanediol diglycidyl ether (BDDE), which is used in the majority of market-leading HA hydrogels.

### HA crosslinking with BDDE

A preliminary experiment was done by cross-linking through mixing 823 kDa HA 2,5 % (m/v), dissolved in 0.1 M NaOH by overnight stirring, with 5 and 10% (v/v) BDDE in glass jars. The reaction was conducted for 48 h until no more increase in solution viscosity was observed. HA solutions were observed before and after the cross-linking.

Before cross-linking, all three solutions were liquid. After 24 h, gelation of HA solution containing 10% BDDE was observed, and after 48 h, both 5% and 10% BDDE-containing solutions were cross-linked, while HA without BDDE remained liquid.

### HA hydrogels produced by drop deposition, HA pre-mixed with BDDE

The inventors selected HA 823 kDa, which seemed to absorb more PAR30-rhodamine than other HAs.

They tested the method which consists in dissolving 5% HA 823 kDa in NaOH 0.25 M and pre-mixing it with BDDE 10% or 20%.

Deposition was tested on 12 mm diameter glass slides.

The glass slides were first washed in Hellmanex 2% solution, then in HCl 1 M (both steps followed by rinsing in demineralized water), then rinsed in ethanol 70% and dried. To improve HA adhesion, a layer of polyethyleneimine (PEI) was deposited by immersion of the glass slides in 0.5 mg/ml PEI solution in water for 30 minutes.

The mix was deposited on the prepared glass slides, and the plates containing the slides were sealed to avoid film drying **(****Figure 1****).** The cross-linking reaction was conducted for 48h.

HA dissolving in NaOH allows increasing the HA concentration up to 5% without the solution being too viscous. Pre-mixing BDDE with HA allows using smaller quantities of the cross-linker.

Layers obtained by deposition of 25 µL HA 5%, BDDE 10% were about 800 µm thick and homogeneous. When 10 µL HA 5%-BDDE 10% were deposited and spread on the slide, the film thicknesses decreased to approximately 250 µm. Finally, when 5 µL HA 5% + BDDE 20% were deposited between two glass slides, 50 µm film thicknesses were obtained.

Thus, deposition of HA pre-mixed with BDDE allows obtainin films of different thicknesses, depending on the deposited volume and deposition method.

At the end, the inventors selected HA 2.5% + 20% BDDE (pre-mixed), 10 µL deposition between parafilm and glass slide **(****Figure 2****)** which gave about 100 µm homogeneous films.

### PAR pre-loading vs. post-loading

Next, the inventors tested PAR-charged HA hydrogels for antibacterial activity. They assessed post-loading of PAR (adding 1 mg/mL solution of PAR onto cross-linked HA films). PAR loaded were of 4 different lengths: 10, 30, 150 and 200 residues, further referred to as PAR10, PAR30, PAR150 and PAR200. We also followed PAR release after 24h from post-loaded films.

The antibacterial activity was tested using *S*. *aureus* culture (400 µL of bacterial suspension with an initial optical density OD=0.001 per well of a 24-well plate containing or not HA-covered, PAR charged or not glass slides). After 24h, OD at 620 nm was measured and bacterial viability on the surfaces was assessed using BacLight Redox Sensor CTC Vitality kit (Molecular Probes) as a fluorescent marker.

The results showed inhibition of bacterial growth on the films post-loaded with PAR (all lengths).

The inventors identified the PAR post-loading method as the most efficient.

### EXAMPLE 2: Production and characterization of free-standing HA hydrogels

In the first time, the inventors set up a protocol to produce thin hydrogel films by mixing 823 kDa HA 2.5% (w/v) and 1.4-butanediol diglycidyl ether (BDDE) 20% (v/v) in NaOH 0.25 M and depositing the solution between parafilm and 12-mm diameter glass slide.

This approach gave about 100 µm thin films, but required using of parafilm, which is temperature-sensitive and can affect hydrogel formation. To avoid the lack of reproducibility, the inventors developed a new approach which allowed to produce free-standing hydrogels of different sizes, resistant and easy to manipulate.

### Construction of free-standing HA hydrogels

To prepare such hydrogels, 1.5 mL of 2.5% HA and 20% BDDE well-mixed solution in NaOH 0.25 M was poured into a 35-mm diameter Petri dish and allowed to cross-link at room temperature for 72 h.

The hydrogel was further cut into the discs of required size using a circle cutter, e.g. for the experiments in 24-well plates, 4 mm diameter discs were used.

The hydrogel discs were further rinsed in Tris 10 mM/NaCl 0.15 M buffer (pH = 7.4) and could be kept at 4°C for several weeks.

The schematic protocol for hydrogel disc preparation and the resulting discs of different sizes are shown in **Figure 4****.** The resulting hydrogels can be easily manipulated with a pincer or spatula.

### EXAMPLE 3: PAR loading and release characterization

### Loading of PAR into HA hydrogel discs

To load PAR into the hydrogels, the discs were immersed in PAR solution and incubated at room temperature. For 4 mm discs in 24-well plate, 0.5 mL of PAR solution was used. Then the discs were rinsed with Tris/NaCl buffer two times: one short and one long (at least 1 hour) rinsing.

The procedure, as well as an example of a resulting PAR30-FITC (PAR having 30 arginine residues and conjugated to FITC) loaded disc, are shown in **Figure 5****.**

The inventors compared loading of PAR30 for 3 h *vs.* 24 h. The results showed that after 24 h, PAR30 diffused more into the discs center **(****Figure 6****).** Hence, 24h loading was selected for further experiments.

They next studied loading of three different PARs: PAR10, corresponding to chains having 10 arginine residues; PAR30, corresponding to chains having 30 arginine residues and PAR200, corresponding to chains having 200 arginine residues.

To visualize PAR loading and diffusion inside the hydrogels, they again used fluorescently-labelled PARs (PAR10-FITC, PAR30-FITC, PAR200-FITC).

Fluorescent profiles showed more homogeneous distribution for PAR10 and PAR30 **(****Figure 7****).**

Next, the inventors studied PARs mobility inside the hydrogels **(****Figure 8****)** using FRAP (fluorescence recovery after photobleaching) technique. Qualitatively, PAR10 was the most mobile and PAR200 was the least mobile **(****Figure 8A****).** Quantitative parameters such as diffusion coefficient were also determined **(****Figure 8B****).**

### PAR-loaded hydrogel stability in Tris/NaCl buffer

HA discs loaded with three different PARs (PAR10, corresponding to chains having 10 arginine residues; PAR30, corresponding to chains having 30 arginine residues 30 and PAR200 corresponding to chains having 200 arginine residues) were incubated for 48 h at room temperature or at 37°C. The results showed no PAR-FITC release in any of the conditions, suggesting that antibacterial HA-PAR discs remain stable in Tris/NaCl buffer even at higher temperature, which is good for discs manipulation and transportation.

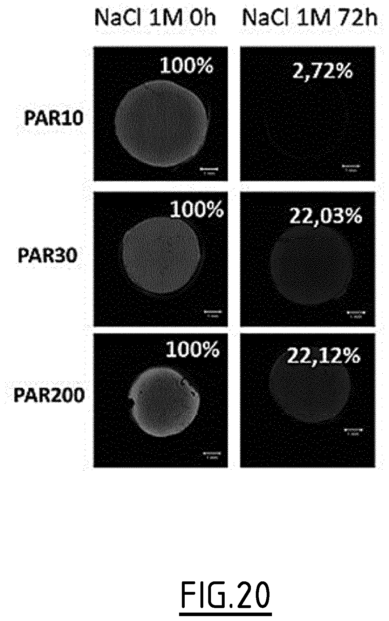

More specifically, total amounts of PAR contained in the hydrogels were estimated by incubation of hydrogels loaded with fluorescently labelled PAR in concentrated NaCl to promote PAR release. After 72h at 37°C in NaCl 1M, the release was almost complete for PAR10 and close to 80% for PAR30 and PAR200, according to the confocal microscopy images **(****Figure 20****).** The percentage of PAR remaining in the hydrogel discs after 72h of incubation was measured with image processing; 100% corresponds to fluorescence intensity before release. Then, percentage of remaining PAR after NaCl 1M incubation was determined to obtain a value of released PAR: about 97%, 78% and 78% for PAR10, PAR30 and PAR200, respectively. Incomplete release of PAR30 and PAR200 correlates with lower mobility demonstrated by FRAP experiments. Then, amounts of PAR-FITC released were quantified by measuring fluorescence intensity of the supernatant by spectrofluorimetry and referring to a calibration curve.

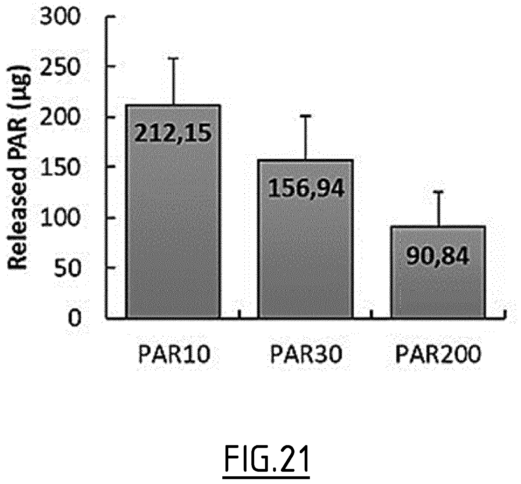

The results show that the discs incubated in 0.5 mg.mL⁻¹ PAR solutions released about 212 µg of PAR10-FITC, 157 µg of PAR30-FITC and 91 µg of PAR200-FITC after 72h in NaCl 1M **(****Figure 21****).** When correcting the released quantities to 100%, it gives 218 µg, 201 µg and 117 µg of loaded PAR10-FITC, PAR30-FITC and PAR200-FITC, respectively. Discs volume is approximately 30 µL, so the discs contain about 7.3 mg.mL⁻¹ of PAR10, 6.7 mg.mL⁻¹ of PAR30 and 3.9 mg.mL⁻¹ of PAR200, respectively.

### PAR release in bacterial and cell culture media

Using FITC-labeled PARs of 10 units, 30 units and 200, the inventors evaluated their release at 37°C from HA hydrogels in two different media.

They performed discs imaging after 24 h of incubation at 37°C in MH (Mueller Hinton broth, bacterial culture medium) and in DMEM (Dulbecco's modified Eagle's medium + 10% FBS + antibiotics, cell culture medium). The results showed that PAR release patterns were slightly different in MH and DMEM/FBS. In the latter, release of PAR30 and PAR200 was higher than in MH.

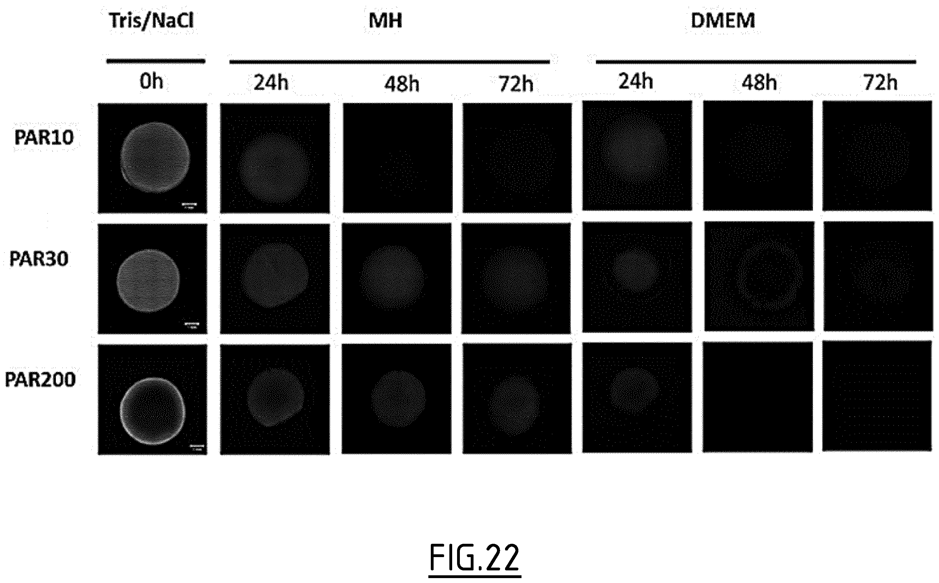

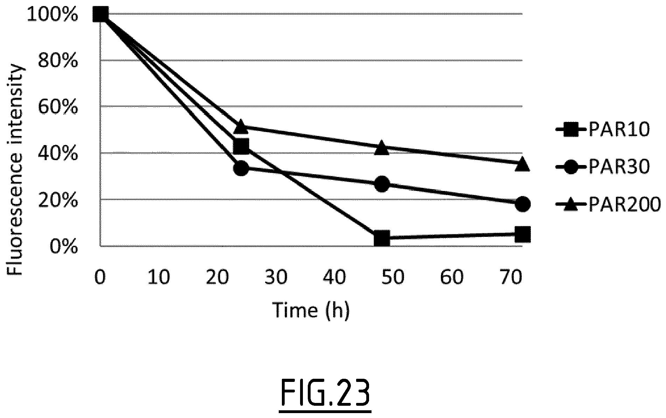

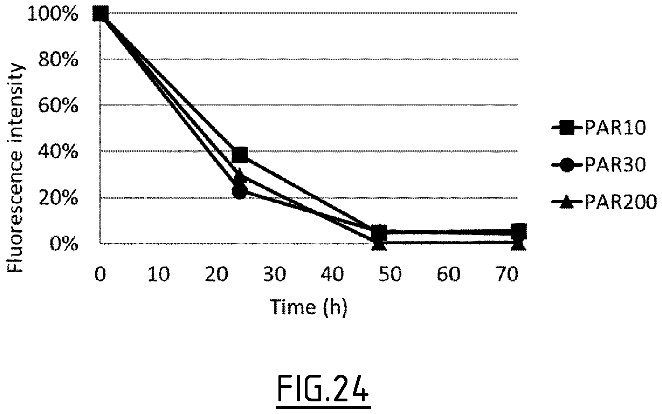

More specifically, PAR release from the hydrogels was followed during 72 hours in microbiological growth medium (MH) or cell culture medium (DMEM). PAR-FITC loaded hydrogels were placed into these media and incubated at 37°C, and PAR release was observed by confocal microscopy **(****Figure 22****).** The release was faster for PAR10 in MH, compared to PAR30 and PAR200, which were released more gradually **(****Figure 23****).** In DMEM, all three PAR had more or less similar release profiles and were completely released after 48h **(****Figure 24****).**

### EXAMPLE 4: Antibacterial effect vs. in vitro cytotoxicity

In the preliminary experiments, the inventors demonstrated antibacterial activity of PAR10, PAR30 and PAR200 loaded into HA hydrogels at 1 mg/mL.

To study antibacterial properties of PAR-loaded hydrogels in more details, they performed repetitive culture **(****Figure 9****)** of bacteria with hydrogel discs loaded with different concentrations of PAR10, PAR30 and PAR200.

PAR30 had the most prolonged antibacterial effect, remaining efficient at 0.5 and 1 mg/mL (loaded) after 8 days of repetitive culture. PAR10 remained efficient for 8 days at 1 mg/mL, and PAR200 remained efficient for 7 days at 1 mg/mL **(****Figures 10-12****).**

### Cytotoxicity tests

Direct *in vitro* cytotoxicity test consists in putting material in contact with the cells for 24 h and performing MTT test to evaluate cell viability. According to ISO 10993, the tested material should cover approximately 1/10 of cell layer surface, which corresponds to ~5 mm hydrogel discs per well of a 24-well plate.

The inventors used 4 mm diameter discs which swelled and became ~6 mm diameter when they were placed in Tris-NaCl buffer.

After 24 h at 37°C, hydrogel discs were removed and MTT test was performed in order to measure cell metabolic activity, which often serves to estimate cell viability. Yellow water-soluble MTT (3-(4,5-dimethylthiazol-2-yl)-2,5-diphenyltetrazoliumbromid) is metabolically reduced in viable cells to a blue-violet insoluble formazan. The number of viable cells correlates to the colour intensity determined by photometric measurements after dissolving the formazan in alcohol (from ISO 10993).

So the discs, loaded or not with PAR, were sterilized and placed onto -80% confluent layer of Balb/3T3 cells. For MTT assay, the cells were incubated for 2 h in 0.2 mg/mL MTT-containing cell culture medium. The medium was then removed and formazan was dissolved in DMSO. Absorbance of resulting solutions was measured at 570 nm using spectrophotometer and images were taken around and under the discs to evaluate cell morphology.

In first experiments, hydrogels loaded with 0.05 mg/mL PAR10 and PAR30 (lowest loading concentration showing antibacterial effect) appeared to be non-cytotoxic by ISO 10993 norms, according to quantitative MTT test results (good viability) and qualitative reactivity gradation (reactivity limited to area under specimen) **(****Figure 13****).**

The inventors confirmed these results and, in addition, performed cytotoxicity assay on PAR10 and PAR30 0.1 mg/mL-loaded hydrogels, which also appeared to be non-cytotoxic **(****Figure 14****).** As previously, reactivity zone was limited to the area under the discs (data not shown) and cell viability was good **(Fig. 20B).** PAR200-loaded hydrogels, however, were classed as cytotoxic.

Of note, PAR10 and PAR30 0.1 mg/mL-loaded hydrogels showed 2 and 4 days of antibacterial effect in repetitive culture (=fresh bacteria added every day). In an additional test **(****Figure 15****),** these numbers were even higher (3 and 5 days).

### EXAMPLE 5: Deposition of HA hydrogels on mesh materials

In addition to setting up a protocol for free-standing hydrogel disc production, the inventors attempted hydrogel deposition onto two materials used for clinical applications: a non-woven fabric used for wounds desinfection, with high absorption power (Medicomp^{®}) and polypropylene mesh for hernia repair.

HA-BDDE solution (50 or 100 µL) was deposited on 12-mm diameter fabric or mesh pieces and allowed to cross-link. Medicomp^{®} absorbed and retained 100 µL of hydrogel solution, while 50 µL amount was more suitable for polypropylene meshes which are not absorbent. However, both materials were able to retain the hydrogels after cross-linking, and easy to manipulate.

Confocal images of PAR30-rhodamine loaded HA hydrogels deposited onto Medicomp^{®} fabric and polypropylene meshes were obtained. In these images, fabric or mesh fibers are surrounded by PAR30-rhodamine labeled hydrogels. Some PAR30-rhodamine is also adsorbed on the fibers.

In terms of antibacterial activity, hydrogel-coated mesh materials were compared to hydrogel discs and showed similar bacterial growth inhibition at low concentration **(****Figure 16****)** for 6 days.

### EXAMPLE 6: Storage and sterilization

The hydrogels (free-standing, as well as deposited onto mesh materials) can be kept for several days at 4°C, dried, frozen and sterilized by autoclaving **(****Figure 17****)** without losing antibacterial activity.

### EXAMPLE 7: Cross-linking percentage

Hydrogels with lower or higher cross-linking degrees (10% and 30% BDDE v/v) loaded with PAR30 showed similar antibacterial activity after 24h, as compared to 20% BDDE **(****Figure 18****).** However, they were more difficult to handle: 10% BDDE hydrogels were very soft and elastic, while 30% BDDE hydrogels were fragile and broke easily.

### EXAMPLE 8: Use of other positively charged antibacterial polypeptides

After 24 h, HA hydrogels loaded with 0.05 and 0.1 mg/mL of polyornithine PLO30 and polylysine PLL30 showed similar antibacterial activity, as compared to PAR30-loaded hydrogels **(****Figure 19****).**

### EXAMPLE 9: In vivo biocompatibility

Ten 8-week-old male Wistar rats (300-400g in weight), provided by a certified breeding centre (Charles River, France) were used for this study.

The animals were received at the CREFRE (US 006/CREFRE - Inserm/UPS/ENVT) animal supplier (No. A31555010 issued December 17, 2015). Protocols were submitted to the CREFRE ethics committee with approval, in accordance with the European directive (DE 86/ 609/CEE; modified DE 2003/65/CE) for conducting animal experiments. One week of acclimatization was respected. The animals were housed in ventilated cages with a double level (two animals per cage according to European standards). The animals were carefully monitored (behavior and food intake) and were weighed weekly throughout the experiment. The 10 rats received each 2 round implants (diameter of 1 cm), one implant on left side and one implant on right side. In total, there were 5 implantations of dried and autoclaved hydrogels deposited onto mesh materials for each of the following conditions: i) HA-only hydrogels; ii) HA hydrogels loaded with PAR10 at 0.1 mg.mL⁻¹; iii) HA hydrogels loaded with PAR30 at 0.05 mg.mL⁻¹; iv) HA hydrogels loaded with PAR30 at 0.1 mg.mL⁻¹.

The rats were induced by isoflurane 4% and maintenance of 2%. Each rat was placed in a prone position on a heated pad. After shaving and scrubbing with betadine, two 20 mm dorsal incisions were made over the thoracolumbar area, one on the right side and one on the left side. One scaffold was inserted at both sides into subcutaneous pockets. All the incisions were closed with Vicryl^{®} 3-0. All rats received buprenorphine (0.6 mg/kg) injected subcutaneously twice per day for 5 days. All animals survived the duration of the study with no adverse effects. Euthanasia were performed after 14 days. The animals were first anesthetized with isoflurane device and mask and then slowly injected with an overdose of pentobarbital (150 mg/kg) in intraperitoneal route. After the expiration of the animal death, the implants with surrounding tissue were explanted and collected to perform histology.

For histological analysis, the samples were fixed in 4% formalin. Macroscopic sections were embedded in paraffin. Five-µm thick sections were stained with hematoxylin-eosin-saffron (HES). For each sample, microscopic optical analysis was realized with the software NDP.view2 (Hamamatsu, Massy, France) after slides scanning (NanoZoomer, Hamamatsu) with the following criteria: semi-quantitative assessment of acute inflammation, chronic inflammation, fibroblastic reaction, edema, fibrosis, angiogenesis and periprosthetic histiocytic reaction.

### Results

Preliminary *in vivo* experiments were conducted on rats (10 animals). Each rat received 2 implants of hydrogel-coated meshes (d = 1 cm), one implant on left side and one implant on right side. All animals survived the duration of the study with no adverse effects and all animals gained weight in a normal way.

After 14 days, the implants with surrounding tissue were explanted and collected to perform histological analysis. The results of the analysis showed the presence of inflammation in the tissues surrounding the implants. However, there was no difference between HA-only hydrogels and HA-PAR hydrogels, suggesting that PAR addition does not promote or increase inflammatory response.

### CONCLUSION

In summary, the inventors developed hyaluronic acid (HA) hydrogels that can be loaded with polyarginine (PAR) and provide a long lasting antibacterial effect. This effect is dependent on the concentration and length of loaded PAR. PAR30 was identified as the most efficient in providing a prolonged antibacterial effect, which increases with PAR concentration.

The antibacterial hydrogels can be deposited onto wound dressings and mesh prosthesis and may help to prevent infections, thus improving tissue regeneration and/or implant integration.

## Claims

1. A hydrogel comprising hyaluronic acid (HA) or a derivative thereof, loaded with at least one positively charged antimicrobial peptide, without said peptide being covalently linked to the hydrogel, wherein said HA or derivative thereof is cross-linked with a cross-linking agent at the level of its hydroxyl moieties while the carboxyl moieties of HA or derivative thereof remain free and said HA or derivative thereof remains negatively charged, wherein the derivatives of hyaluronic acid are selected from the group consisting of: HA modified with an aldehyde group, amine-modified hyaluronic acid, HA containing photoreactive vinylbenzyl groups, HA modified with a methacrylate group, HA conjugated to Tyramine, and HA conjugated to catechol.

2. Hydrogel according to claim 1, wherein said positively charged antimicrobial peptide is selected from the group consisting of polyarginine, polyornithine and polylysine.

3. Hydrogel according to claim 1 or 2, wherein said positively charged antimicrobial peptide is polyarginine.

4. Hydrogel according to claim 3, wherein said polyarginine is of the following formula (1) 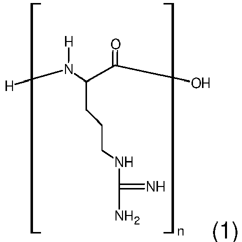 wherein n is an integer comprising between 2 and 250.

5. Hydrogel according to claim 4, wherein said polyarginine is of the following formula (1) 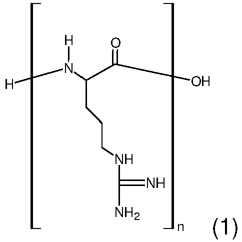 wherein n is 30.

6. Hydrogel according to any one of claims 1 to 5, wherein said hyaluronic acid is hyaluronic acid having a molecular weight of between 800 and 850 kDa.

7. Hydrogel according to any one of claims 1 to 6, wherein said cross-linking agent is butanediol diglycidyl ether (BDDE).

8. Method for preparing a hydrogel according to any one of claims 1 to 7, wherein said method comprises the following steps:
(a) mixing, in basic conditions, hyaluronic acid (HA) or a derivative thereof with a cross-linking agent which cross-links HA at the level of its hydroxyl moieties while the carboxyl moieties of HA or derivative thereof remain free and said HA or derivative thereof remains negatively charged, wherein the derivatives of hyaluronic acid are selected from the group consisting of: HA modified with an aldehyde group, amine-modified hyaluronic acid, HA containing photoreactive vinylbenzyl groups, HA modified with a methacrylate group, HA conjugated to Tyramine, and HA conjugated to catechol
(b) depositing the mixture on a support and incubating it for 48 h to 72 h at room temperature to obtain a hydrogel,
(c) recovering the hydrogel formed at step (b),
(d) incubating said hydrogel in an aqueous buffer in conditions enabling the withdrawal of cross-linking agent residues and the hydrogel to swell,
(e) loading the hydrogel obtained at step (d) with at least one positively charged antimicrobial peptide, without said peptide being covalently linked to the hydrogel, and
(f) recovering the loaded hydrogel obtained at step (e).

9. Method according to claim 8, wherein the mixture of step (a) comprises from 2 to 3 % (w/v) of HA or derivative thereof, and at least 10% (v/v) of cross-linking agent, in particular at least 20% (v/v) of cross-linking agent.

10. Method according to claim 8 or 9, wherein said cross-linking agent is butanediol diglycidyl ether (BDDE).

11. Method according to any one of claims 8 to 10, wherein said positively charged antimicrobial peptide is polyarginine.

12. Method according to any one of claims 8 to 11, wherein said positively charged antimicrobial peptide is loaded at step (e) at a concentration of 0.05 to 1 mg/ml.

13. Hydrogel according to claim 1, obtained by the method of preparation according to any one of claims 8 to 12.

14. Medical device comprising a hydrogel according to any one of claims 1 to 7 or 13.

15. Medical device according to claim 14, wherein said medical device is a wound dressing or a mesh prosthesis.

## Patentansprüche

1. Hydrogel, umfassend Hyaluronsäure (HA) oder ein Derivat davon, beladen mit mindestens einem positiv geladenen antimikrobiellen Peptid, ohne dass das Peptid kovalent an das Hydrogel gebunden ist, wobei die HA oder das Derivat davon mit einem Vernetzungsmittel auf der Ebene ihrer Hydroxylanteile vernetzt ist, während die Carboxylanteile der HA oder des Derivats davon frei bleiben und die HA oder das Derivat davon negativ geladen bleibt, wobei die Derivate der Hyaluronsäure ausgewählt sind aus der Gruppe bestehend aus: HA, modifiziert mit einer Aldehydgruppe, HA, aminmodifizierte Hyaluronsäure, HA, die photoreaktive Vinylbenzylgruppen enthält, HA, die mit einer Methacrylatgruppe modifiziert ist, HA, die mit Tyramin konjugiert ist, und HA, die mit Catechol konjugiert ist.

2. Hydrogel nach Anspruch 1, wobei das positiv geladene antimikrobielle Peptid ausgewählt ist aus der Gruppe bestehend aus Polyarginin, Polyornithin und Polylysin.

3. Hydrogel nach Anspruch 1 oder 2, wobei das positiv geladene antimikrobielle Peptid Polyarginin ist.

4. Hydrogel nach Anspruch 3, wobei das Polyarginin von der folgenden Formel (1) ist 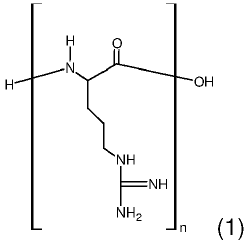 wobei n eine ganze Zahl ist, umfassend zwischen 2 bis 250.

5. Hydrogel nach Anspruch 4, wobei das Polyarginin von der folgenden Formel (1) ist 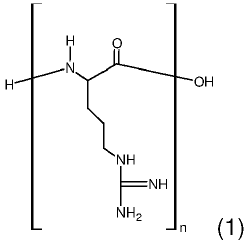 wobei n 30 ist.

6. Hydrogel nach einem der Ansprüche 1 bis 5, wobei die Hyaluronsäure Hyaluronsäure ist, die ein Molekulargewicht zwischen 800 und 850 kDa aufweist.

7. Hydrogel nach einem der Ansprüche 1 bis 6, wobei das Vernetzungsmittel Butandioldiglycidylether (BDDE) ist.

8. Verfahren zum Herstellen eines Hydrogels nach einem der Ansprüche 1 bis 7, wobei das Verfahren die folgenden Schritte umfasst:
(a) Mischen, unter basischen Bedingungen, von Hyaluronsäure (HA) oder einem Derivat davon mit einem Vernetzungsmittel, das die HA auf Ebene ihrer Hydroxylgruppen vernetzt, während die Carboxylgruppen der HA oder des Derivats davon frei bleiben und die HA oder das Derivat davon negativ geladen bleibt, wobei die Derivate der Hyaluronsäure ausgewählt sind aus der Gruppe bestehend aus: HA, modifiziert mit einer Aldehydgruppe, HA, aminmodifizierte Hyaluronsäure, HA, die photoreaktive Vinylbenzylgruppen enthält, HA, die mit einer Methacrylatgruppe modifiziert ist, HA, die mit Tyramin konjugiert ist, und HA, die mit Catechol konjugiert ist,
(b) Aufbringen des Gemischs auf einen Träger und Inkubieren über 48 bis 72 Stunden bei Raumtemperatur, um ein Hydrogel zu erlangen,
(c) Gewinnen des in Schritt (b) gebildeten Hydrogels,
(d) Inkubieren des Hydrogels in einem wässrigen Puffer unter Bedingungen, die den Entzug von Vernetzungsmittelresten und ein Quellen des Hydrogels ermöglichen,
(e) Beladen des in Schritt (d) erlangten Hydrogels mit mindestens einem positiv geladenen antimikrobiellen Peptid, ohne dass das Peptid kovalent mit dem Hydrogel verbunden ist, und
(f) Gewinnen des in Schritt (e) erlangten beladenen Hydrogels.

9. Verfahren nach Anspruch 8, wobei das Gemisch aus Schritt (a) 2 bis 3 % (Gewicht/Volumen) HA oder ein Derivat davon und mindestens 10 % (Volumen/Volumen) Vernetzungsmittel, insbesondere mindestens 20 % (Volumen/Volumen) Vernetzungsmittel, umfasst.

10. Verfahren nach Anspruch 8 oder 9, wobei das Vernetzungsmittel Butandioldiglycidylether (BDDE) ist.

11. Verfahren nach einem der Ansprüche 8 bis 10, wobei das positiv geladene antimikrobielle Peptid Polyarginin ist.

12. Verfahren nach einem der Ansprüche 8 bis 11, wobei das positiv geladene antimikrobielle Peptid in Schritt (e) in einer Konzentration von 0,05 bis 1 mg/ml geladen wird.

13. Hydrogel nach Anspruch 1, erlangt durch das Verfahren zum Herstellen nach einem der Ansprüche 8 bis 12.

14. Medizinische Vorrichtung, umfassend ein Hydrogel nach einem der Ansprüche 1 bis 7 oder 13.

15. Medizinische Vorrichtung nach Anspruch 14, wobei die medizinische Vorrichtung ein Wundverband oder eine Netzprothese ist.

## Revendications

1. Hydrogel comprenant de l'acide hyaluronique (AH) ou un dérivé de celui-ci, chargé d'au moins un peptide antimicrobien chargé positivement, sans que ledit peptide ne soit lié de manière covalente à l'hydrogel, dans lequel ledit AH ou dérivé de celui-ci est réticulé avec un agent de réticulation au niveau de ses moitiés hydroxyle tandis que les moitiés carboxyle de l'AH ou du dérivé de celui-ci restent libres et ledit AH ou dérivé de celui-ci reste chargé négativement, dans lequel les dérivés de l'acide hyaluronique sont choisis dans le groupe constitué de : un AH modifié par un groupe aldéhyde, un acide hyaluronique modifié par une amine, un AH contenant des groupes vinylbenzyle photoréactifs, un AH modifié par un groupe méthacrylate, un AH conjugué à la tyramine et un AH conjugué au catéchol.

2. Hydrogel selon la revendication 1, dans lequel ledit peptide antimicrobien chargé positivement est choisi dans le groupe constitué par la polyarginine, la polyornithine et la polylysine.

3. Hydrogel selon la revendication 1 ou 2, dans lequel ledit peptide antimicrobien chargé positivement est la polyarginine.

4. Hydrogel selon la revendication 3, dans lequel ladite polyarginine est de la formule suivante (1) 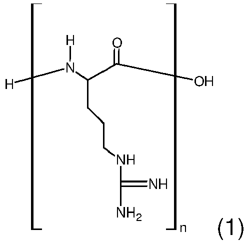 dans lequel n est un nombre entier compris entre 2 et 250.

5. Hydrogel selon la revendication 4, dans lequel ladite polyarginine est de la formule suivante (1) 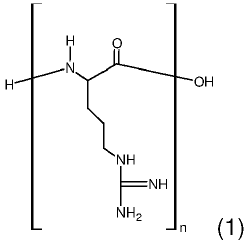 dans lequel n est égal à 30.

6. Hydrogel selon l'une quelconque des revendications 1 à 5, dans lequel ledit acide hyaluronique est un acide hyaluronique doté d'un poids moléculaire compris entre 800 et 850 kDa.

7. Hydrogel selon l'une quelconque des revendications 1 à 6, dans lequel l'agent de réticulation est l'éther diglycidylique de butanediol (BDDE).

8. Procédé de préparation d'un hydrogel selon l'une quelconque des revendications 1 à 7, dans lequel ledit procédé comprend les étapes suivantes :
(a) le mélange, dans des conditions basiques, d'acide hyaluronique (AH) ou d'un dérivé de celui-ci avec un agent de réticulation qui réticule l'AH au niveau de ses moitiés hydroxyle tandis que les moitiés carboxyle de l'AH ou du dérivé de celui-ci restent libres et que ledit AH ou dérivé de celui-ci reste chargé négativement, dans lequel les dérivés de l'acide hyaluronique sont choisis dans le groupe constitué par : un AH modifié par un groupe aldéhyde, un acide hyaluronique modifié par une amine, un AH contenant des groupes vinylbenzyle photoréactifs, un AH modifié par un groupe méthacrylate, un AH conjugué à la tyramine et un AH conjugué au catéchol
(b) le dépôt du mélange sur un support et son incubation pendant 48 h à 72 h à la température ambiante pour obtenir un hydrogel,
(c) la récupération de l'hydrogel formé à l'étape (b),
(d) l'incubation dudit hydrogel dans un tampon aqueux dans des conditions permettant le retrait des résidus de l'agent de réticulation et le gonflement de l'hydrogel,
(e) la charge de l'hydrogel obtenu à l'étape (d) avec au moins un peptide antimicrobien chargé positivement, sans que ledit peptide ne soit lié de manière covalente à l'hydrogel, et
(f) la récupération de l'hydrogel chargé obtenu à l'étape (e).

9. Procédé selon la revendication 8, dans lequel le mélange de l'étape (a) comprend de 2 à 3 % (en poids) d'AH ou de ses dérivés, et au moins 10 % (en volume) d'agent de réticulation, en particulier au moins 20 % (en volume) d'agent de réticulation.

10. Procédé selon la revendication 8 ou 9, dans lequel l'agent de réticulation est l'éther diglycidylique de butanediol (BDDE).

11. Procédé selon l'une quelconque des revendications 8 à 10, dans lequel ledit peptide antimicrobien chargé positivement est la polyarginine.

12. Procédé selon l'une quelconque des revendications 8 à 11, dans lequel ledit peptide antimicrobien chargé positivement est chargé à l'étape (e) à une concentration de 0,05 à 1 mg/ml.

13. Hydrogel selon la revendication 1, obtenu par le procédé de préparation selon l'une quelconque des revendications 8 à 12.

14. Dispositif médical comprenant un hydrogel selon l'une quelconque des revendications 1 à 7 ou 13.

15. Dispositif médical selon la revendication 14, dans lequel ledit dispositif médical est un pansement ou une prothèse à mailles.
